(19) Europäisches Patentamt / European Patent Office / Office européen des brevets

(11) **EP 2 292 274 A1**

(12) **EUROPEAN PATENT APPLICATION**

(43) Date of publication:
**09.03.2011 Bulletin 2011/10**

(51) Int Cl.:
***A61K 48/00*** (2006.01) ***C12N 15/63*** (2006.01)

(21) Application number: **10183733.4**

(22) Date of filing: **08.09.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.09.2004 US 610853 P**
**15.03.2005 US 661841 P**

(62) Document number(s) of the earlier application(s) in accordance with Art. 76 EPC:
**05794863.0 / 1 789 095**

(71) Applicant: **Sangamo BioSciences, Inc.**
**Richmond, CA 94804 (US)**

(72) Inventors:
- **Li, Xiao-Yong**
  **Albany, CA, 94706 (US)**
- **Jamieson, Andrew**
  **San Francisco, CA, 94115 (US)**
- **Bartsevich, Victor**
  **Albany, CA, 94706 (US)**
- **Collingwood, Trevor**
  **Novato, CA, 94947 (US)**

(74) Representative: **Weiss, Wolfgang et al**
**Weickmann & Weickmann**
**Postfach 860820**
**81635 München (DE)**

Remarks:
This application was filed on 30-09-2010 as a divisional application to the application mentioned under INID code 62.

(54) **Compositions and methods for protein production**

(57) Disclosed herein are methods and compositions for enhanced protein production and overexpression using engineered zinc finger proteins.

**FIGURE 7**

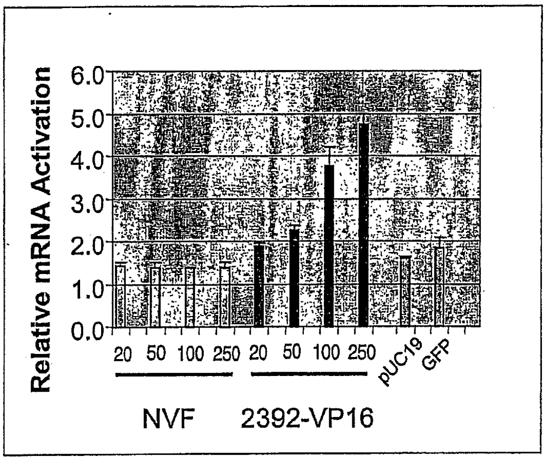




Printed by Jouve, 75001 PARIS (FR)

## Description

CROSS-REFERENCE TO RELATED APPLICATIONS

This application claims the benefit of United States provisional patent applications No. 60/610,853 (filed September 16, 2004) and 60/661,841 (filed March 15, 2005) the disclosures of which are incorporated by reference in their entireties for all purposes.

STATEMENT OF RIGHTS TO INVENTIONS

MADE UNDER FEDERALLY SPONSORED RESEARCH

Not applicable.

TECHINICAL FIELD

The present disclosure is in the field of transcriptional regulation, particularly protein expression and overproduction, for example, large-scale production of therapeutic proteins.

BACKGROUND

Controlled regulation of transcription is useful in the fields of research, diagnostics and therapeutics. For example, regulation of transcription of a nucleotide sequence encoding a protein can facilitate production of large amounts of recombinant protein for use as a therapeutic, for screening, for lead optimization and for target validation.

Typical expression systems are characterized by a host cell line comprising an expression vector containing a heterologous promoter operatively linked to a cDNA encoding a gene product of interest. One such system for protein expression (and overexpression) uses the SRα promoter, which is composed of a fusion between the SV40 early promoter and the R segment and part of the U5 sequence form the long terminal repeat of human T-cell leukemia virus type 1. Takebe et al. (1988) Mol. Cell. Biol. 8:466-472. Another overexpression system utilizes a human cytomegalovirus (CMV) immediate early promoter. U.S. Patents 5,168,062 and 5,385,839. Both of these promoters are regulated by endogenous, naturally-occurring transcription factors, whose availability or activity may limit the amount of transcription, and hence the amount of protein produced, in these systems. Moreover, overexpression of the naturally-occurring transcription factors that regulate these promoters could lead to aberrant expression of genes normally regulated by these factors, with potential detrimental effects on expression of the desired gene product.

Additional methods for protein production involve integration of promoters or other regulatory sequences into a chromosome adj acent to a gene whose expression is to be regulated. See, *e.g.*, U.S. Patents 5,272,071; 5,641,670; 5,733,761; 5,968,502 and 6,361,972. These, too, depend of the action of endogenous transcription factors and thus are subject to the potential limitations discussed above for the SRα and CMV systems. Moreover, they also suffer from difficulties in achieving precisely targeted chromosomal integration of exogenous polynucleotides.

Expression systems which yield levels of protein that are higher than those obtained by using the SRα and CMV promoters, and which do not depend on random integration of exogenous polynucleotide sequences, would be desirable. Moreover, expression systems utilizing exogenous transcription factors allow the design of customized transcription factors, provide greater flexibility and provide the potential to obtain higher levels of expression of gene products of interest.

SUMMARY

In one aspect, a method for regulating the transcription of a nucleotide sequence in a cell is provided, the method comprising expressing, in the cell, a protein that binds to a target site comprising SEQ ID NO:1, wherein SEQ ID NO:1 is operatively linked to the nucleotide sequence. In certain embodiments, a plurality of target sites are operatively linked to the nucleotide sequence.

In another aspect, described herein is a method for regulating transcription of first and second nucleotide sequences in a cell, the method comprising expressing, in the cell, a protein that binds to a target site comprising SEQ ID NO:1 and wherein SEQ ID NO:1 is operatively linked to the first and second nucleotide sequences. A plurality of target sites may be operatively linked the first nucleotide sequence, to the second nucleotide sequence or to both the first and second nucleotide sequences.

In any of them methods described herein, the protein may comprise, for example, SEQ ID NO:25 or equivalents thereof (e.g., SEQ ID NO:2, SEQ ID NO:3, SEQ ID NO:7, SEQ ID NO:8). Thus, described herein are methods for regulating transcription of a first (and/or second) nucleotide sequence(s) in a cell by expressing SEQ ID NO:25 or an equivalent in the cell. In certain embodiments, the protein further comprises a transcriptional activation domain (*e.g.*, VP16).

In any of the methods described herein, the nucleotide sequence can encode a mRNA that is translated to yield one or more protein(s). Furthermore, in any of the methods described herein, the nucleotide sequence(s) may comprises a cDNA sequence, for example one or more cDNA sequence encoding antibody polypeptides (e.g., antibody heavy chain or light chain). In additional embodiments, the nucleotide sequence can encode a RNA molecule that is not translated into protein such as, for example, siRNA, micro RNA, rRNA, tRNA, snRNA or scRNA.

In another aspect, described herein are methods for regulating the transcription of a nucleotide sequence in a cell, the method comprising expressing, in the cell, a protein comprising SEQ ID NO:25, SEQ ID NO:2, SEQ ID NO:3, SEQ ID NO:7, SEQ ID NO:8 or equivalents, wherein the protein binds to a target site; wherein the target site is operatively linked to the nucleotide sequence. In certain embodiments, the target site comprises SEQ ID NO:1. One or more target sites may be operatively linked to the nucleotide sequence. In certain embodiments, the nucleotide sequence comprises a cDNA sequence. The protein may further comprise a transcriptional activation domain, for example a VP16 domain.

In yet another aspect, described herein is a method for regulating the transcription of first and second nucleotide sequences in a cell, the method comprising: expressing, in the cell, a protein comprising SEQ ID NO:25, SEQ ID NO: 2, SEQ ID NO:3, SEQ ID NO:7, SEQ ID NO:8 or equivalents, wherein the protein binds to a target site; wherein the target site is operatively linked to both of the first and second nucleotide sequences. In certain embodiments, the target site comprises SEQ ID NO:1. One or more target sites may be operatively linked to the first and/or second nucleotide sequences. In certain embodiments, the first and/or nucleotide sequence comprises a cDNA sequence, for example a cDNA sequence encoding antibody polypeptides (*e.g.*, antibody light chains and/or antibody heavy chains). The protein may further comprise a transcriptional activation domain, for example a VP16 domain.

In yet another aspect, polypeptides comprising SEQ ID NO:25 are provided. In certain embodiments, the polypeptides comprise SEQ ID NO:2 or SEQ ID NO:7 or equivalents thereof.

In a still further aspect, polynucleotides encoding any of the polypeptides disclosed herein are provided.

In yet another aspect, cells comprising any of the polypeptides and/or polynucleotides disclosed herein are provided.

In another aspect, provided herein are vectors comprising SEQ ID NO:1; cells comprising any of these vectors as well as a cell comprising one or more copies of SEQ ID NO:1 integrated into its genome.

These and other embodiments will be readily apparent to one of skill in the art upon reading the present disclosure.

## BRIEF DESCRIPTION OF THE DRAWINGS

**Figure 1** shows the nucleotide sequence (SEQ ID NO:9) of a hybrid SV40-R-U5 promoter (SR$\alpha$ promoter), synthesized as described in Example 1. The target site for the various ZFPs described in Example 2 is underlined.

**Figure 2** shows the amino acid sequence of the 2392/00 protein (SEQ ID NO:10). The domains in the protein are as follows. Amino acids 3-9: nuclear localization sequence; amino acids 15-109: zinc finger domain; amino acids 119-185: VP 16 transcriptional activation domain; amino acids 196-203: FLAG epitope tag.

**Figure 3** shows the sequence of a polynucleotide (SEQ ID NO:11) encoding the 2392/00 protein. Portions of the sequence encoding the various domains in the protein are as follows. Nucleotides 7-27: nuclear localization sequence; nucleotides 43-327: zinc finger domain; nucleotides 355-555: VP16 transcriptional activation domain; nucleotides 586-609: FLAG epitope tag.

**Figure 4** shows the amino acid sequence of the 2392/10 protein (SEQ ID NO:12). The domains in the protein are as follows. Amino acids 3-9: nuclear localization sequence; amino acids 15-109: zinc finger domain; amino acids 119-185: VP16 transcriptional activation domain; amino acids 196-203: FLAG epitope tag.

**Figure 5** shows the sequence of a polynucleotide (SEQ ID NO:13) encoding the 2392/10 protein. Portions of the sequence encoding the various domains in the protein are as follows. Nucleotides 7-27: nuclear localization sequence; nucleotides 43-327: zinc finger domain; nucleotides 355-555: VP16 transcriptional activation domain; nucleotides 586-609: FLAG epitope tag.

**Figure 6** shows the amino acid sequences of a number of three-finger zinc finger domains that were obtained from a two-hybrid selection system to recognize the target sequence GCTGTGGAA (SEQ ID NO:1). See Example 2 for details.

**Figure 7** shows levels of immunoglobulin kappa chain mRNA in cells containing an integrated transcription unit comprising a SR$\alpha$ promoter and kappa chain cDNA. Cells were transfected with plasmids encoding a VP 16 activation domain (NVF) or the 2393/00 ZFP fused to the VP16 activation domain (2392-VP16). Numbers on the abscissa refer to nanograms of DNA transfected. See Example 3 for details.

**Figure 8** shows levels of secreted antibody in two cell lines (A and B) transfected with a plasmid encoding the 2392/00 ZFP-VP16 fusion protein (2392), compared to cells transfected with a plasmid encoding the VP16 activation domain (NVF). GFP: cells transfected with a plasmid encoding a green-fluorescent protein; Mock: mock-transfected cells; ntf: non-transfected cells. See Example 3 for details.

**Figure 9** shows levels of immunoglobulin gamma heavy chain and immunoglobulin kappa light chain mRNA in cells containing amplified gamma and kappa chain cDNAs, both under the transcriptional control of the SRα promoter ("High Producer Line" in Figure). Results from cells transfected with a plasmid encoding the 2392/00-VP16 fusion protein are indicated by "ZFP" along the abscissa; non-transfected cells are indicated by "NT." mRNA levels were normalized to those of GAPDH. See Example 3 for details.

**Figure 10** shows levels of immunoglobulin G secreted from cells stably transfected with sequences encoding the 2392/00-VP16 fusion protein (labeled "ZFP" in the Figure). Secreted IgG levels from untransfected cells (labeled "Control") are also shown.

**Figure 11** shows relative IgG levels in cells transfected with two different plasmids containing both a heavy chain-encoding transcription unit and a light chain-encoding transcription unit. In one of the plasmids, each transcription unit is under the transcriptional control of a SRα promoter (denoted SRa in the figure). In the other plasmid, each transcription unit is under the transcriptional control of a SRα promoter to which 8 additional copies of SEQ ID NO: 1 have been appended (denoted SRa in the figure). 2392/10-7 refers to a clonal isolate of CHO cells stably transfected with a nucleic acid encoding the 2392/10-VP16 fusion protein. DG44 refers to the parental, untransfected CHO cell line.

**Figure 12** shows levels of green fluorescent protein mRNA (normalized to GAPDH mRNA) in 2392/10-7 cells, which contain sequences encoding a ZFP-VP16 fusion protein. Cells were transfected with plasmids containing various modified CMV promoters operatively linked to sequences encoding green fluorescent protein (GFP). The number of copies of target site SEQ ID NO:1 inserted adj acent to the CMV promoter in each construct is shown below the graph, as is the orientation of the inserted target sites. The rightmost bar in each pair shows GFP mRNA levels in the ZFP-containing cell line; the leftmost bar shows GFP mRNA levels in a parental cell line that does not express the ZFP.

**Figure 13** shows levels of erythropoietin (Epo) secreted from cells transfected with different Epo expression constructs. SRαZ6 refers to a construct in which Epo expression is controlled by a SRα promoter containing seven copies of SEQ ID NO:1. CMV refers to a construct in which Epo expression is controlled by a CMV promoter. CMVz10 refers to a construct in which Epo expression is controlled by a CMV promoter containing ten copies of SEQ ID NO: 1. The rightmost bar in each pair shows levels of Epo secreted from the ZFP-containing cell line 2392/10-7; the leftmost bar show levels of Epo secreted from a parental cell line that does not express the ZFP. See Example 9 for details.

## DETAILED DESCRIPTION

### General

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See,* for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P.M. Wassarman and A. P. Wolffe, eds.), Academic Press, San Diego, 1999; and METHODS IN MOLECULAR BIOLOGY, Vol. 119, "Chromatin Protocols" (P.B. Becker, ed.) Humana Press, Totowa, 1999.

### Definitions

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (e.g., phosphorothioate backbones). In general, an analogue of a particular nucleotide has the same base-pairing specificity; i.e., an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

Techniques for determining nucleic acid and amino acid sequence identity are known in the art. Typically, such techniques include determining the nucleotide sequence of the mRNA for a gene and/or determining the amino acid sequence of a protein encoded by a gene or mRNA, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, Nucl. Acids Res. 14 (6):6745-6763 (1986). An exemplary implementation of this algorithm to determine percent identity of a sequence is provided by the Genetics Computer Group (Madison, WI) in the "BestFit" utility application. The default parameters for this method are described in the Wisconsin Sequence Analysis Package Program Manual, Version 8 (1995) (available from Genetics Computer Group, Madison, WI). A preferred method of establishing percent identity in the context of the present disclosure is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects sequence identity. Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect =10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST. With respect to sequences described herein, the range of desired degrees of sequence identity is approximately 20% to 100% and any integer value therebetween. Typically the percent identities between sequences are at least 70-75%, preferably 80-82%, more preferably 85-90%, even more preferably 92%, still more preferably 95%, and most preferably 98% sequence identity. Alternatively, the degree of sequence similarity between polynucleotides can be determined by hybridization of polynucleotides under conditions that allow formation of stable duplexes between homologous regions, followed by digestion with single-stranded-specific nuclease(s), and size determination of the digested fragments, Two nucleic acid, or two polypeptide sequences are substantially homologous to each other when the sequences exhibit at least about 70%-75%, preferably 80%-82%, more preferably 85%-90%, even more preferably 92%, still more preferably 95%, and most preferably 98% sequence identity over a defined length of the molecules, as determined using the methods above. As used herein, substantially homologous also refers to sequences showing complete identity to a specified DNA or polypeptide sequence. DNA sequences that are substantially homologous can be identified in a Southern hybridization experiment under, for example, stringent conditions, as defined for that particular system. Defining appropriate hybridization conditions is within the skill of the art. See, *e.g.*, Sambrook et al., *supra;* Nucleic Acid Hybridization: A Practical Approach, editors B.D. Hames and S.J. Higgins, (1985) Oxford; Washington, DC; IRL Press).

Selective hybridization of two nucleic acid fragments can be determined as follows. The degree of sequence identity between two nucleic acid molecules affects the efficiency and strength of hybridization events between such molecules. A partially identical nucleic acid sequence will at least partially inhibit the hybridization of a completely identical sequence to a target molecule. Inhibition of hybridization of the completely identical sequence can be assessed using hybridization assays that are well known in the art (*e.g.*, Southern (DNA) blot, Northern (RNA) blot, solution hybridization, or the like, see Sambrook, et al., Molecular Cloning: A Laboratory Manual, Second Edition, (1989) Cold Spring Harbor, N.Y.). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a reference nucleic acid sequence, and then by selection of appropriate conditions the probe and the reference sequence selectively hybridize, or bind, to each other to form a duplex molecule. A nucleic acid molecule that is capable of hybridizing selectively to a reference sequence under moderately stringent hybridization conditions typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/reference sequence hybridization, where the probe and reference sequence have a specific degree of sequence identity, can be determined as is known in the art (see, for example, Nucleic Acid Hybridization: A Practical Approach, editors B.D. Hames and S.J. Higgins, (1985) Oxford; Washington, DC; IRL Press).

Conditions for hybridization are well-known to those of skill in the art. Hybridization stringency refers to the degree to which hybridization conditions disfavor the formation of hybrids containing mismatched nucleotides, with higher stringency correlated with a lower tolerance for mismatched hybrids. Factors that affect the stringency of hybridization are well-known to those of skill in the art and include, but are not limited to, temperature, pH, ionic strength, duration of the hybridization reaction and concentration of organic solvents such as, for example, formamide and dimethylsulfoxide. As is known to those of skill in the art, hybridization stringency is increased by higher temperatures, lower ionic strength and lower solvent concentrations.

With respect to stringency conditions for hybridization, it is well known in the art that numerous equivalent conditions can be employed to establish a particular stringency by varying, for example, the following factors: the length and nature of the sequences, base composition of the various sequences, concentrations of salts and other hybridization solution components, the presence or absence of blocking agents in the hybridization solutions (e.g., dextran sulfate, and polyethylene glycol), hybridization reaction temperature and time parameters, as well as, varying wash conditions. The selection of a particular set of hybridization conditions is selected following standard methods in the art (see, for example, Sambrook, et al., Molecular Cloning: A Laboratory Manual, Second Edition, (1989) Cold Spring Harbor, N.Y.).

"Binding" refers to a sequence-specific, non-covalent interaction between macromolecules (*e.g.*, between a protein and a nucleic acid). Not all components of a binding interaction need be sequence-specific (*e.g.*, contacts with phosphate residues in a DNA backbone), as long as the interaction as a whole is sequence-specific. Such interactions are generally characterized by a dissociation constant ($K_d$) of $10^{-6}$ $M^{-1}$ or lower. "Affinity" refers to the strength of binding: increased binding affinity being correlated with a lower $K_d$.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, *etc.*) and/or it can bind to one or more molecules of a different protein or proteins. A binding protein can have more than one type of binding activity. For example, zinc finger proteins have DNA-binding, RNA-binding and protein-binding activity.

A "zinc finger DNA binding protein" (or zinc finger binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP.

Zinc finger binding domains can be "engineered" to bind to a predetermined nucleotide sequence. Non-limiting examples of methods for engineering zinc finger proteins are design and selection.

A designed zinc finger protein is a protein not occurring in nature whose design/composition results principally from rational criteria. Rational criteria for design include application of substitution rules and computerized algorithms for processing information in a database storing information of existing ZFP designs and binding data. See, for example, US Patents 6,140,081; 6,453,242; and 6,534,261; see also WO 98/53058; WO 98/53059; WO 98/53060; WO 02/016536 and WO 03/016496.

A "selected" zinc finger protein is a protein not found in nature whose production results primarily from an empirical process such as phage display, interaction trap or hybrid selection. See *e.g.*, US 5,789,538; US 5,925,523; US 6,007,988; US 6,013,453; US 6,200,759; WO 95/19431; WO 96/06166; WO 98/53057; WO 98/54311; WO 00/27878; WO 01/60970 WO 01/88197 and WO 02/099084.

Thus, an "engineered zinc finger protein" refers to a protein that contains one or more zinc fingers, that has been constructed to bind in a sequence-specific fashion to a predetermined nucleotide sequence. Generally, engineered zinc finger proteins are non-naturally-occurring proteins and/or contain naturally-occurring zinc fingers in non-naturally-occurring arrangements and/or combinations. Methods for engineering the binding specificity of zinc fingers and constructing engineered zinc finger proteins include, but are not limited to, rational design, randomization/selection techniques, polysome selection, cis-display, one- and two-hybrid systems, and selection from randomized libraries of both engineered and naturally-occurring zinc fingers. *See,* for example, U.S. Patents 5,789,538; 6,007,988; 6,013,453; 6,140,466; 6,242,568; 6,410,248; 6,453,242; 6,479,626; 6,503,717; 6,534,261; 6,706,470; 6,733,970; and 6,746,838; U.S. Patent Application publications 2003/0044957; 2003/0068675; 2003/0104526; 2003/0108880; 2003/0166141 and 2004/0091991; and PCT Publications WO 98/53057; WO 98/53058; WO 98/53059; WO 98/53060; WO 01/53480; WO 01/88197; and WO 02/77227, the disclosures of which are incorporated herein by reference in their entireties for all purposes. The term "engineered zinc finger protein" does not refer to a cloned, naturally-occurring zinc finger protein.

"Chromatin" is the nucleoprotein structure comprising the cellular genome. Cellular chromatin comprises nucleic acid, primarily DNA, and protein, including histones and non-histone chromosomal proteins. The majority of eukaryotic cellular chromatin exists in the form of nucleosomes, wherein a nucleosome core comprises approximately 150 base pairs of DNA associated with an octamer comprising two each of histones H2A, H2B, H3 and H4; and linker DNA (of variable length depending on the organism) extends between nucleosome cores. A molecule of histone H1 is generally associated with the linker DNA. For the purposes of the present disclosure, the term "chromatin" is meant to encompass all types of cellular nucleoprotein, both prokaryotic and eukaryotic. Cellular chromatin includes both chromosomal and episomal chromatin.

A "chromosome," is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An "episome" is a replicating nucleic acid, nucleoprotein complex or other structure comprising a nucleic acid that is not part of the chromosomal karyotype of a cell. Examples of episomes include plasmids and certain viral genomes.

A "target site" or "target sequence" is a nucleic acid sequence that defines a portion of a nucleic acid to which a binding molecule (*e.g.*, a binding protein) will bind, provided sufficient conditions for binding exist. For example, the sequence 5'-GAATTC-3' is a target site for the Eco RI restriction endonuclease.

An "accessible region" is a site in cellular chromatin in which a target site present in the nucleic acid can be bound by an exogenous molecule which recognizes the target site. Without wishing to be bound by any particular theory, it is believed that an accessible region is one that is not packaged into a nucleosomal structure. The distinct structure of an accessible region can often be detected by its sensitivity to chemical and enzymatic probes, for example, nucleases.

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Patent Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, *e.g.*, an exogenous protein or nucleic acid. For example, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (*i.e.*, liposomes, including neutral and cationic lipids), electroporation, direct injection, cell fusion, particle bombardment, calcium phosphate co-precipitation, DEAE-dextran-mediated transfer and viral vector-mediated transfer.

By contrast, an. "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

A "fusion" molecule is a molecule in which two or more subunit molecules are linked, preferably covalently. The subunit molecules can be the same chemical type of molecule, or can be different chemical types of molecules: Examples of the first type of fusion molecule include, but are not limited to, fusion proteins (for example, a fusion between a ZFP DNA-binding domain and a transcriptional regulatory domain) and fusion nucleic acids (for example, a nucleic acid encoding the fusion protein described *supra).* Examples of the second type of fusion molecule include, but are not limited to, a fusion between a triplex-forming nucleic acid and a polypeptide, and a fusion between a minor groove binder and a nucleic acid.

Expression of a protein (e.g., a fusion protein) in a cell can result from delivery of the protein to the cell or by delivery of a polynucleotide encoding the protein to a cell, wherein the polynucleotide is transcribed, and the transcript is translated, to generate the protein. Delivery to the cell of a RNA molecule, which is subsequently translated in the cell, can also be used to express a protein in a cell. Trans-splicing, polypeptide cleavage and polypeptide ligation can also be involved in expression of a protein in a cell. Methods for polynucleotide and polypeptide delivery to cells are known in the art and exemplary methods are presented elsewhere in this disclosure.

A "gene," for the purposes of the present disclosure, includes a DNA region encoding a gene product (see *infra),* as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information, contained in a gene, into a gene product. A gene product can be the direct transcriptional product of a gene (e.g., mRNA, tRNA, rRNA, antisense RNA, dsRNA, ribozyme, structural RNA or any other type of RNA), a processed transcript such as, for example siRNA, or a protein produced by translation of a mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the activity of a gene. Modulation of expression can include, but is not limited to, gene activation and gene repression.

"Eucaryotic" cells include, but are not limited to, fungal cells (such as yeast), plant cells, animal cells, mammalian cells and human cells.

A "region of interest" is any region of cellular chromatin, such as, for example, a gene or a non-coding sequence within or adjacent to a gene, in which it is desirable to bind an exogenous molecule. Binding can be for the purposes of, *e.g.*, transcriptional regulation. A region of interest can be present in a chromosome, an episome, an organellar genome (*e.g.*, mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region. A region of interest can be as small as a single nucleotide pair or up to 2,000 nucleotide pairs in length, or any integral value of nucleotide pairs.

The terms "operative linkage" and "operatively linked" (or "operably linked") are used interchangeably with reference to a juxtaposition of two or more components (such as sequence elements), in which the components are arranged such that the components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in *cis* with a coding sequence, but need not be directly adjacent to it. For example, an enhancer is a transcriptional regulatory sequence that is operatively linked to a coding sequence, even though they are not contiguous.

With respect to fusion polypeptides, the term "operatively linked" can refer to the fact that each of the components performs the same function in linkage to the other component as it would if it were not so linked. For example, with respect to a fusion polypeptide in which a ZFP DNA-binding domain is fused to a transcriptional regulatory domain, the ZFP DNA-binding domain and the regulatory domain are in operative linkage if, in the fusion polypeptide, the ZFP DNA-binding domain portion is able to bind its target site and/or its binding site, while the regulatory domain is able to modulate (e.g., activate or repress) transcription.

A "functional equivalent" or "functional fragment" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one ore more amino acid or nucleotide substitutions. Methods for determining the function of a nucleic acid (e.g., coding function, ability to hybridize to another nucleic acid) are well-known in the art. Similarly, methods for determining protein function are well-known. For example, the DNA-binding function of a polypeptide can be determined, for example, by filter-binding, electrophoretic mobility-shift, or immunoprecipitation assays. DNA cleavage can be assayed by gel electrophoresis. See Ausubel *et al., supra.* The ability of a protein to interact with another protein can be determined, for example, by co-immunoprecipitation, two-hybrid assays or complementation, both genetic and biochemical. See, for example, Fields et al. (1989) Nature 340: 245-246; U.S. Patent No. 5,585,245 and PCT WO 98/44350.

## Engineered Zinc Finger Proteins and Target Sequences

Disclosed herein are compositions and methods for regulation of transcription, which are useful, for example, for enhanced production of RNA and/or protein. These include fusion proteins comprising an engineered zinc finger protein and a functional domain such as, for example, a transcriptional activation domain. Suitable functional domains are known in the art and include, without limitation, transcriptional activation domains such as, for example, VP16, VP64 and p65. Moreover, one or more of the same or different functional domains (*e.g.*, transcriptional activation domains) can be present in a given fusion protein. *See* co-owned U.S. Patent Application Publication No. 2002/0160940, incorporated by reference, for disclosure of exemplary functional domains.

In certain embodiments, a zinc finger protein is engineered to bind to a sequence comprising the target sequence GCTGTGGAA (SEQ ID NO:1). This sequence is present in the SR$\alpha$ promoter (Takebe *et al., supra),* a promoter commonly used for protein production, but one or more copies SEQ ID NO:1 can be inserted in or adjacent to any promoter known in the art (e.g., a CMV promoter).

An exemplary three-finger zinc finger protein, SBS2392/00, that has been engineered to bind to SEQ ID NO: 1 has the amino acid sequence:

KKKQHICHIQGCGKVYGQRSNLVRHLRWHTGERPFMCTWSYCGKRFTRSDA LSRHKRTHTGEKKFACPECPKRFMQSSDLRRHIKTHQNK (SEQ ID NO:2).

The underlined amino acid residues in SEQ ID NO:2 correspond to residues - 1 through +6 with respect to the start of the alpha-helical portion of a zinc finger and are denoted the "recognition regions" because one or more of these residues participate in sequence specificity of nucleic acid binding. Accordingly, proteins comprising the same three recognition regions in a different polypeptide backbone sequence are considered equivalents to the protein identified as SEQ ID NO:2, since they will have the same DNA-binding specificity.

Thus, in certain embodiments, the three recognition regions (underlined in SEQ ID NO:2 above) can be placed in any zinc finger backbone (see, *e.g.*, U.S. Patents 6,453,242 and 6,534,261) and the resulting protein can be used to regulate transcription, e.g., to enhance protein production. Accordingly, engineered zinc finger proteins having the following sequence can be used in the disclosed methods: C-$X_{2-4}$-C-$X_5$-QRSNL VR-H-$X_{3-5}$-H-$X_7$-C-$X_{2-4}$-C-$X_5$-RSDALSR-H-$X_{3-5}$-H-$X_7$-C-$X_{2-4}$-C-$X_5$-QSSDLRR-H-$X_{3-5}$-H (SEQ ID NO:3).

Within the recognition region, residues -1, +3 and +6 are primarily responsible for protein-nucleotide contacts. Accordingly, non-limiting examples of additional equivalents include proteins comprising three zinc fingers wherein the first finger contains a Q residue at -1, a N residue at +3 and a R residue at +6 (QXXNXXR, SEQ ID NO:4); the second finger contains a R residue at -1, an A residue at +3 and a R residue at +6 (RXXAXXR, SEQ ID NO:5); and the third finger contains a Q residue at -1, a D residue at +3 and a R residue at +6 (QXXDXXR, SEQ ID NO:6). Additional equivalents comprise any ZFP that binds to a sequence comprising the target sequence GCTGTGGAA (SEQ ID NO:1).

An additional exemplary three-finger zinc finger protein engineered to bind the target sequence GCTGTGGAA (SEQ ID NO:1), SBS2392/10, has the following amino acid sequence:

KKKQHICHIQGCGKVYGQSSNLARHLRWHTGERPFMCTWSYCGKRF TRSDALTRHKRTHTGEKKFACPECPKRFMQSCDLTRHIKTHQNK (SEQ ID NO:7)The underlined amino acid residues in SEQ ID NO:7 correspond to residues -1 through +6 with respect to the start of the alpha-helical portion of a zinc finger and are denoted the "recognition regions" because one or more of these residues participate in sequence specificity of nucleic acid binding. Accordingly, proteins comprising the same three recognition regions in a different polypeptide backbone sequence are considered equivalents to SEQ ID NO:7, since they will have the same DNA-binding specificity.

Thus, in certain embodiments, the three recognition regions (underlined in SEQ ID NO:7 above) can be placed in any zinc finger backbone (see, *e.g.*, U.S. Patents 6,453,242 and 6,534,261) and the resulting protein can be used to regulate transcription, *e.g.*, to enhance protein production. Accordingly, engineered zinc finger proteins having the following sequence can be used in the disclosed methods:

C-$X_{2-4}$-C-$X_5$-QSSNLAR-H-$X_{3-5}$-H-$X_7$-C-$X_{2-4}$-C-$X_5$-RSDALTR-H-$X_{3-5}$-H-$X_7$-C-$X_{2-4}$-C-$X_5$-QSCDLTR-H-$X_{3-5}$-H (SEQ ID NO:8).

Within the recognition region, residues -1, +3 and +6 are primarily responsible for protein-nucleotide contacts. Accordingly, non-limiting examples of additional equivalents include proteins comprising three zinc fingers wherein the first finger contains a Q residue at -1, a N residue at +3 and a R residue at +6 (QXXNXXR, SEQ ID NO:4); the second finger contains a R residue at -1, an A residue at +3 and a R residue at +6 (RXXAXXR, SEQ ID NO:5); and the third finger contains a Q residue at -1, a D residue at +3 and a R residue at +6 (QXXDXXR, SEQ ID NO: 6). Thus, for example, proteins comprising SEQ ID NO:25 are considered equivalents for use in the disclosed methods.

$$C\text{-}X_{2\text{-}4}\text{-}C\text{-}X_5\text{-}QXXNXXR\text{-}H\text{-}X_{3\text{-}5}\text{-}H\text{-}X_7\text{-}C\text{-}X_{2\text{-}4}\text{-}C\text{-}X_5\text{-}RXXAXXR\text{-}H\text{-}X_{3\text{-}5}\text{-}H\text{-}X_7\text{-}C\text{-}X_{2\text{-}4}\text{-}C\text{-}X_5\text{-}QXXDXXR\text{-}H\text{-}X_{3\text{-}5}\text{-}H \quad \text{(SEQ ID NO:25)}$$

Additional equivalents comprise any ZFP that binds to a sequence comprising the target sequence GCTGTGGAA (SEQ ID NO:1).

Correspondences between amino acids at the -1, +3 and +6 contact residues of the recognition region of a zinc finger, and nucleotides in a target site, have been described. See, for example; U.S. Patent Nos. 6,007,988; 6,013,453 and 6,746,838; as well as PCT Publications WO 96/06166; WO 98/53058; WO 98/53059 and WO 98/53060. Accordingly, also to be considered equivalents are three-finger zinc finger proteins in which the first finger contains Q at -1; N at +3 and R, K, S or T at +6; the second finger contains R at -1; A, S or V at +3 and R, K, S or T at +6; and the third finger contains N, Q, H or T at -1; S, D, E, L, T, or V at +3 and R, K, S or T at +6.

## Fusion proteins and encoding polynucleotides

As previously stated, the engineered zinc finger DNA-binding domains disclosed herein can comprise a portion of a fusion protein, wherein the fusion protein also contains one or more functional domains (e.g., transcriptional regulatory domains), nuclear localization sequences, epitope tags, *etc.* For example, the amino acid sequence of a fusion protein comprising a nuclear localization sequence (NLS), a zinc finger binding domain (ZFP), the VP16 transcriptional activation domain (VP16) and a FLAG epitope tag (FLAG), denoted 2392/00, is shown in Figure 2 (SEQ ID NO:10). The nucleotide sequence encoding this protein is shown in Figure 3 (SEQ ID NO:11).

An additional exemplary protein (2392/10) has the amino acid sequence (SEQ ID NO:12) shown in Figure 4. The sequence of a polynucleotide (SEQ ID NO:13) encoding the 2392/10 protein is shown in Figure 5.

## Functional Domains

Any DNA-binding domain can optionally be associated with one or more "functional domains" or "regulatory domains" which facilitate, *e.g.*, DNA processing (e.g., DNA cleavage) or modulation of gene expression. The binding domain can be covalently or non-covalently associated with one or more regulatory domains, alternatively two or more regulatory domains, with the two or more domains being two copies of the same domain, or two different domains. The regulatory domains can be covalently linked to the binding domain, *e.g.*, via an amino acid linker, as part of a fusion protein. A DNA-binding domain can also be associated with a regulatory domain via a non-covalent dimerization domain, *e.g.*, a leucine zipper, a STAT protein N terminal domain, or an FK506 binding protein *(see, e.g.,* O'Shea, Science 254: 539 (1991), Barahmand-Pour et al., Curr. Top. Microbiol. Immunol. 211:121-128 (1996); Klemm et al., Annu. Rev. Immunol. 16:569-592 (1998); Klemm et al., Annu. Rev. Immunol. 16:569-592 (1998); Ho et al., Nature 382:822-826 (1996); and Pomeranz et al., Biochem. 37:965 (1998)). The regulatory domain can be associated with the binding domain at any suitable position, including the C- or N-terminus of the binding domain.

Common regulatory domains include, e.g., effector domains from transcription factors (activators, repressors, co-activators, co-repressors), silencers, nuclear hormone receptors, oncogene transcription factors (e.g., myc, jun, fos, myb, max, mad, rel, ets, bcl, myb, mos family members etc.); DNA repair enzymes and their associated factors and modifiers; DNA rearrangement enzymes and their associated factors and modifiers; chromatin associated proteins and their modifiers (*e.g.*, kinases, acetylases and deacetylases); and DNA modifying enzymes (*e.g.*, methyltransferases, topoisomerases, helicases, ligases, kinases, phosphatases, polymerases, endonucleases, integrases) and their associated factors and modifiers.

Transcription factor polypeptides from which one can obtain a regulatory domain include those that are involved in regulated and basal transcription. Such polypeptides include transcription factors, their effector domains, coactivators, silencers, nuclear hormone receptors (*see, e.g.,* Goodrich et al., Cell 84:825-30 (1996) for a review of proteins and nucleic acid elements involved in transcription; transcription factors in general are reviewed in Barnes & Adcock, Clin. Exp. Allergy 25 Suppl. 2:46-9 (1995) and Roeder, Methods Enzymol. 273:165-71 (1996)). Databases dedicated to transcription factors are known *(see, e.g.,* Science 269:630 (1995) and TRANSFAC). Nuclear hormone receptor transcription factors are described in, for example, Rosen et al., J. Med. Chem. 38:4855-74 (1995). The C/EBP family of transcription factors are reviewed in Wedel et al., Immunobiology 193:171-85 (1995). Coactivators and co-repressors that mediate transcription regulation by nuclear hormone receptors are reviewed in, for example, Meier, Eur. J. Endocrinol. 134(2):158-9 (1996); Kaiser et al., Trends Biochem. Sci. 21:342-5 (1996); and Utley et al., Nature 394:498-502 (1998)). GATA transcription factors, which are involved in regulation of hematopoiesis, are described in, for example, Simon, Nat. Genet. 11:9-11 (1995); Weiss et al., Exp. Hematol. 23:99-107. TATA box binding protein (TBP) and its associated TAF polypeptides (which include TAF30, TAF55, TAF80, TAF110, TAF150, and TAF250) are described in Goodrich & Tjian, Curr. Opin. Cell Biol. 6:403-9 (1994) and Hurley, Curr. Opin. Struct. Biol. 6:69-75 (1996). The STAT family of transcription factors are reviewed in, for example, Barahmand-Pour et al., Curr. Top. Microbiol. Immunol. 211:121-8 (1996). Transcription factors involved in disease are reviewed in Aso et al., J Clin. Invest. 97:1561-9 (1996).

In one embodiment, the KOX repression domain and/or the KRAB repression domain from the human KOX-1 protein is used as a transcriptional repressor. Thiesen et al., New Biologist 2:363-374 (1990); Margolin et al., PNAS 91: 4509-4513 (1994); Pengue et al., Nucl. Acids Res. 22:2908-2914 (1994); Witzgall et al., PNAS 91:4514-4518 (1994). In another embodiment, KAP-1, a KRAB co-repressor, is used with KRAB or KOX. Friedman et al., Genes Dev. 10: 2067-2078 (1996). Alternatively, KAP-1 can be used alone as a functional domain. Other preferred transcription factors and transcription factor domains that act as transcriptional repressors include MAD (*see, e.g.,* Sommer et al., J. Biol. Chem. 273:6632-6642 (1998); Gupta et al., Oncogene 16:1149-1159 (1998); Queva et al., Oncogene 16:967-977 (1998); Larsson et al., Oncogene 15:737-748 (1997); Laherty et al., Cell 89:349-356 (1997); and Cultraro et al., Mol Cell. Biol. 17:2353-2359 (19977)); FKHR (forkhead in rhapdosarcoma gene; Ginsberg et al., Cancer Res. 15:3542-3546 (1998); Epstein et al., Mol. Cell. Biol. 18:4118-4130 (1998)); EGR-1 (early growth response gene product-1; Yan et al., PNAS 95:8298-8303 (1998); and Liu et al., Cancer Gene Ther. 5:3-28 (1998)); the ets2 repressor factor repressor domain (ERD; Sgouras et al., EMBO J. 14:4781-4793 ((19095)); and the MAD smSIN3 interaction domain (SID; Ayer et al., Mol. Cell. Biol. 16:5772-5781 (1996)).

In one embodiment, the HSV VP16 activation domain is used as a transcriptional activator (*see, e.g.,* Hagmann et al., J. Virol. 71:5952-5962 (1997)), Other transcription factors from which activation domains can be obtained include nuclear hormone receptors (*see, e.g.,* Torchia et al., Curr. Opin. Cell. Biol. 10:373-383 (1998)); the p65 subunit of nuclear factor kappa B (Bitko & Barik, J. Virol. 72:5610-5618 (1998) and Doyle & Hunt, Neuroreport 8:2937-2942 (1997)); and EGR-1 (early growth response gene product-1; Yan et al., PNAS 95:8298-8303 (1998); and Liu et al., Cancer Gene Ther. 5:3-28 (1998)). An additional synthetic activation domain is the VP64 activation domain (Seipel et al., EMBO J. 11:4961-4968 (1996)).

Kinases, phosphatases, methylases, demethylases, acetylases, deacetylases, and other proteins that modify polypeptides involved in gene regulation are also useful as regulatory domains. Such modifiers are often involved in switching on or off transcription mediated by, for example, hormones. Kinases involved in transcription regulation are reviewed in Davis, Mol. Reprod. Dev. 42:459-67 (1995), Jackson et al., Adv. Second Messenger Phosphoprotein Res. 28:279-86 (1993), and Boulikas, Crit. Rev. Eukaryot. Gene Expr. 5:1-77 (1995), while phosphatases are reviewed in, for example, Schonthal & Semin, Cancer Biol. 6:239-48 (1995). Nuclear tyrosine kinases are described in Wang, Trends Biochem. Sci. 19:373-6 (1994).

As described, useful domains can also be obtained from the gene products of oncogenes (e.g., myc, jun, fos, myb, max, mad, rel, ets, bcl, myb, mos family members) and their associated factors and modifiers. Oncogenes are described in, for example, Cooper, Oncogenes, 2nd ed., The Jones and Bartlett Series in Biology, Boston, MA, Jones and Bartlett Publishers, 1995. The ets transcription factors are reviewed in Waslylk et al., Eur. J. Biochem. 211:7-18 (1993) and Crepieux et al., Crit. Rev. Oncog. 5:615-38 (1994). Myc oncogenes are reviewed in, for example, Ryan et al., Biochem. J. 314:713-21 (1996). The jun and fos transcription factors are described in, for example, The Fos and Jun Families of Transcription Factors, Angel & Herrlich, eds. (1994). The max oncogene is reviewed in Hurlin et al., Cold Spring Harb. Symp. Quant. Biol. 59:109-16. The myb gene family is reviewed in Kanei-Ishii et al., Curr. Top. Microbiol. Immunol. 211:89-98 (1996). The mos family is reviewed in Yew et al., Curr. Opin. Genet. Dev. 3:19-25 (1993).

Regulatory domains can also be obtained from DNA repair enzymes and their associated factors and modifiers. These include, for example, nucleases (exo- and endo-), recombinases, helicases, integrases, polymerases and singie-stranded DNA-binding proteins (SSBs). DNA repair systems are reviewed in, for example, Vos, Curr. Opin. Cell Biol. 4: 385-95 (1992); Sancar, Ann. Rev. Genet. 29:69-105 (1995); Lehmann, Genet. Eng. 17:1-19 (1995); and Wood, Ann. Rev. Biochem. 65:135-67 (1996). DNA rearrangement enzymes and their associated factors and modifiers can also be used as regulatory domains (*see, e.g.,* Gangloff et al., Experientia 50:261-9 (1994); Sadowski, FASEB J. 7:760-7 (1993)).

Similarly, regulatory domains can be derived from DNA modifying enzymes (e.g., DNA methyltransferases, topoisomerases, helicases, ligases, kinases, phosphatases, polymerases) and their associated factors and modifiers. Helicases are reviewed in Matson et al., Bioessays, 16:13-22 (1994), and methyltransferases are described in Cheng, Curr. Opin. Struct, Biol. 5:4-10 (1995). Chromatin associated proteins and their modifiers (*e.g.*, kinases, acetylases and deacetylases), such as histone deacetylase (Wolffe, Science 272:371-2 (1996)) are also useful functional domains. In one embodiment, the regulatory domain is a DNA methyl transferase that acts as a transcriptional repressor *(see, e.g.,* Van den Wyngaert et al., FEBS Lett. 426:283-289 (1998); Flynn et al., J. Mol. Biol. 279:101-116 (1998); Okano et al., Nucleic Acids Res. 26:2536-2540 (1998); and Zardo & Caiafa, J. Biol. Chem. 273:16517-16520 (1998)). In another embodiment, endonucleases such as Fok1 provide functional domains to catalyze targeted DNA cleavage, which facilitates processes such as transcriptional repression and homologous recombination. *See, e.g.,* U. S. Patents 5,436,150; 5,792,640 and 6,265,196; U.S. Patent Application Publication No. 2003/0232410 and WO 03/87341.

Factors that control chromatin and DNA structure, movement and localization and their associated factors and modifiers; factors derived from microbes (e.g., prokaryotes, eukaryotes and virus) and factors that associate with or modify them can also be used to obtain functional domains for the construction of chimeric proteins or fusion molecules. In one embodiment, recombinases and integrases are used as regulatory domains. In another embodiment, histone acetyltransferase is used as a transcriptional activation domain *(see, e.g.,* Jin & Scotto, Mol. Cell. Biol. 18:4377-4384 (1998); Wolffe, Science 272:371-372 (1996); Taunton et al., Science 272:408-411 (1996); and Hassig et al., PNAS 95: 3519-3524 (1998)). In another embodiment, histone deacetylase is used as a transcriptional repression domain *(see, e.g.,* Jin & Scotto, Mol. Cell. Biol, 18:4377-4384 (1998); Syntichaki & Thireos, J. Biol. Chem. 273:24414-24419 (1998); Sakaguchi et al., Genes Dev. 12:2831-2841 (1998); and Martinez et al., J. Biol. Chem. 273:23781-23785 (1998)).

Another suitable repression domain is methyl binding domain protein 2B (MBD-2B) (see, also Hendrich et al. (1999) Mamm Genome 10:906-912 for description of MBD proteins). Another useful repression domain is that associated with the v-ErbA protein. *See,* for example, Damm, et al. (1989) Nature 339:593-597; Evans (1989) Int. J. Cancer Suppl. 4:26-28; Pain et al. (1990) New Biol. 2:284-294; Sap et al. (1989) Nature 340:242-244; Zenke et al. (1988) Cell 52: 107-119; and Zenke et al. (1990) Cell 61:1035-1049. Additional exemplary repression domains include, but are not limited to, thyroid hormone receptor (TR, see *infra*), SID, MBD1, MBD2, MBD3, MBD4, MBD-like proteins, members of the DNMT family (*e.g.*, DNMT1, DNMT3A, DNMT3B), Rb, MeCP1 and MeCP2. *See,* for example, Bird et al. (1999) Cell 99:451-454; Tyler et al. (1999) Cell 99:443-446; Knoepfler et al. (1999) Cell 99:447-450; and Robertson et al. (2000) Nature Genet. 25:338-342. Additional exemplary repression domains include, but are not limited to, ROM2 and AtHD2A. *See,* for example, Chern et al. (1996) Plant Cell 8:305-321; and Wu et al. (2000) Plant J. 22:19-27.

Certain members of the nuclear hormone receptor (NHR) superfamily, including, for example, thyroid hormone receptors (TRs) and retinoic acid receptors (RARs) are among the most potent transcriptional regulators currently known. Zhang et al., Annu. Rev. Physiol. 62:439-466 (2000) and Sucov et al., Mol Neurobiol 10(2-3):169-184 (1995). In the absence of their cognate ligand, these proteins bind with high specificity and affinity to short stretches of DNA (e.g., 12-17 base pairs) within regulatory loci (e.g., enhancers and promoters) and effect robust transcriptional repression of adjacent genes. The potency of their regulatory action stems from the concurrent use of two distinct functional pathways to drive gene silencing: (i) the creation of a localized domain of repressive chromatin *via* the targeting of a complex between the corepressor N-CoR and a histone deacetylase, HDAC3 (Guenther et al., Genes Dev 14:1048-1057 (2000); Urnov et al., EMBO J 19:4074-4090 (2000); Li et al., EMBO J19, 4342-4350 (2000) and Underhill et al., J. Biol. Chem, 275:40463-40470 (2000)) and (ii) a chromatin-independent pathway (Urnov *et al., supra*) that may involve direct interference with the function of the basal transcription machinery (Fondell et al., Genes Dev 7(7B):1400-1410 (1993) and Fondell et al., Mol Cell Biol 16:281-287 (1996).

In the presence of very low (*e.g.*, nanomolar) concentrations of their ligand, these receptors undergo a conformational change which leads to the release of corepressors, recruitment of a different class of auxiliary molecules (*e.g.*, coactivators) and potent transcriptional activation. Collingwood et al., J. Mol. Endocrinol. 23(3):255-275 (1999).

The portion of the receptor protein responsible for transcriptional control (*e.g.*, repression and activation) can be physically separated from the portion responsible for DNA binding, and retains full functionality when tethered to other polypeptides, for example, other DNA-binding domains. Accordingly, a nuclear hormone receptor transcription control domain can be fused to a DNA-binding domain (*e.g.*, a zinc finger protein) such that the transcriptional regulatory activity of the receptor can be targeted to a chromosomal region of interest (e.g., a gene) by virtue of the DNA-binding domain.

Moreover, the structure of TR and other nuclear hormone receptors can be altered, either naturally or through recombinant techniques, such that it loses all capacity to respond to hormone (thus losing its ability to drive transcriptional activation), but retains the ability to effect transcriptional repression. This approach is exemplified by the transcriptional regulatory properties of the oncoprotein v-ErbA. The v-ErbA protein is one of the two proteins required for leukemic transformation of immature red blood cell precursors in young chicks by the avian erythroblastosis virus. TR is a major regulator of erytbropoiesis (Beug et al., Biochim Biophys Acta 1288(3):M35-47 (1996); in particular, in its unliganded state, it represses genes required for cell cycle arrest and the differentiated state. Thus, the administration of thyroid hormone to immature erythroblasts leads to their rapid differentiation. The v-ErbA oncoprotein is an extensively mutated version of TR; these mutations include: (i) deletion of 12 amino-terminal amino acids; (ii) fusion to the *gag* oncoprotein; (iii) several point mutations in the DNA binding domain that alter the DNA binding specificity of the protein relative to its parent, TR, and impair its ability to heterodimerize with the retinoid X receptor; (iv) multiple point mutations in the ligand-binding domain of the protein that effectively eliminate the capacity to bind thyroid hormone; and (v) a deletion of a carboxy-terminal stretch of amino acids that is essential for transcriptional activation. Stunnenberg *et al., Biochim Biophys Acta* 1423(1):F15-33 (1999). As a consequence of these mutations, v-ErbA retains the capacity to bind to naturally occurring TR target genes and is an effective transcriptional repressor when bound (Urnov *et al., supra;* Sap et al.,

Nature 340:242-244 (1989); and Ciana et al., EMBO J. 17(24):7382-7394 (1999). In contrast to TR, however, v-ErbA is completely insensitive to thyroid hormone, and thus maintains transcriptional repression in the presence of thyroid hormones or retinoids.

Accordingly, in one aspect, v-ErbA or its functional fragments are used as a repression domain. In additional embodiments, TR or its functional domains are used as a repression domain in the absence of ligand and/or as an activation domain in the presence of ligand (e.g., 3,5,3'-triiodo-L-thyronine or T3). Thus, TR can be used as a switchable functional domain (i.e., a bifunctional domain); its activity (activation or repression) being dependent upon the presence or absence (respectively) of ligand.

Additional exemplary repression domains are obtained from the DAX protein and its functional fragments. Zazopoulos et al., Nature 390:311-315 (1997). In particular, the C-terminal portion of DAX-1, including amino acids 245-470, has been shown to possess repression activity. Altincicek et al., J. Biol. Chem. 275:7662-7667 (2000). A further exemplary repression domain is the RBP1 protein and its functional fragments. Lai et al., Oncogene 18:2091-2100 (1999); Lai et al., Mol. Cell. Biol. 19:6632-6641 (1999); Lai et al., Mol. Cell. Biol. 21:2918-2932 (2001) and WO 01/04296. The full-length RBP1 polypeptide contains 1257 amino acids. Exemplary functional fragments of RBP1 are a polypeptide comprising amino acids 1114-1257, and a polypeptide comprising amino acids 243-452.

Members of the TIEG family of transcription factors contain three repression domains known as R1, R2 and R3. Repression by TIEG family proteins is achieved at least in part through recruitment of mSIN3A histone deacetylases complexes. Cook et al. (1999) J. Biol. Chem. 274:29,500-29,504; Zhang et al. (2001) Mol. Cell. Biol. 21:5041-5049. Any or all of these repression domains (or their functional fragments) can be fused alone, or in combination with additional repression domains (or their functional fragments), to a DNA-binding domain to generate a targeted exogenous repressor molecule.

Furthermore, the product of the human cytomegalovirus (HCMV) UL34 open reading frame acts as a transcriptional repressor of certain HCMV genes, for example, the US3 gene. LaPierre et al. (2001) J. Virol. 75:6062-6069. Accordingly, the UL34 gene product, or functional fragments thereof, can be used as a component of a fusion polypeptide also comprising a zinc finger binding domain. Nucleic acids encoding such fusions are also useful in the methods and compositions disclosed herein.

Yet another exemplary repression domain is the CDF-1 transcription factor and/or its functional fragments. *See,* for example, WO 99/27092.

The Ikaros family of proteins are involved in the regulation of lymphocyte development, at least in part by transcriptional repression. Accordingly, an Ikaros family member (e.g., Ikaros, Aiolos) or a functional fragment thereof, can be used as a repression domain. *See,* for example, Sabbattini et at. (2001) EMBO J. 20:2812-2822.

The yeast Ashlp protein comprises a transcriptional repression domain. Maxon et al. (2001) Proc. Natl. Acad. Sci. USA 98:1495-1500. Accordingly, the Ashlp protein, its functional fragments, and homologues of Ashlp, such as those found, for example, in, vertebrate, mammalian, and plant cells, can serve as a repression domain for use in the methods and compositions disclosed herein.

Additional exemplary repression domains include those derived from histone deacetylases (HDACs, *e.g.*, Class I HDACs, Class II HDACs, SIR-2 homologues), HDAC-interacting proteins (*e.g.*, SIN3, SAP30, SAP15, NCoR, SMRT, RB, p107, p130, RBAP46/48, MTA, Mi-2, Brg1, Brm), DNA-cytosine methyltransferases (e.g., Dnmt1, Dnmt3a, Dnmt3b), proteins that bind methylated DNA (e.g., MBD1, MBD2, MBD3, MBD4, MeCP2, DMAP1), protein methyltransferases (e.g., lysine and arginine methylases, SuVar homologues such as Suv39H1), polycomb-type repressors (e.g., Bmi-1, eed1, RING1, RYBP, E2F6, Mel18, YY1 and CtBP), viral repressors (e.g., adenovirus E1b 55K protein, cytomegalovirus UL34 protein, viral oncogenes such as v-erbA), hormone receptors (e.g., Dax-1, estrogen receptor, thyroid hormone receptor), and repression domains associated with naturally-occurring zinc finger proteins (e.g., WT1, KAP1). Further exemplary repression domains include members of the polycomb complex and their homologues, HPH1, HPH2, HPC2, NC2, groucho, Eve, tramtrak, mHP1, SIP1, ZEB1, ZEB2, and Enx1/Ezh2. In all of these cases, either the full-length protein or a functional fragment can be used as a repression domain for fusion to a zinc finger binding domain. Furthermore, any homologues of the aforementioned proteins can also be used as repression domains, as can proteins (or their functional fragments) that interact with any of the aforementioned proteins.

Additional repression domains, and exemplary functional fragments, are as follows. Hes1 is a human homologue of the *Drosophila hairy* gene product and comprises a functional fragment encompassing amino acids 910-1014. In particular, a WRPW (trp-arg-pro-trp) motif can act as a repression domain. Fisher et al. (1996) Mol. Cell. Biol.16: 2670-2677.

The TILE1, TLE2 and TLE3 proteins are human homologues of the *Drosophila groucho* gene product. Functional fragments of these proteins possessing repression activity reside between amino acids 1-400. Fisher *et al., supra.*

The Tbx3 protein possesses a functional repression domain between amino acids 524-721. He et al. (1999) Proc. Natl. Acad. Sci. USA 96:10,212-10,217. The Tbx2 gene product is involved in repression of the p 14/p 16 genes and contains a region between amino acids 504-702 that is homologous to the repression domain of Tbx3; accordingly Tbx2 and/or this functional fragment can be used as a repression domain. Carreira et al. (1998) Mol. Cell. Biol. 18:

5,099-5,108.

The human Ezh2 protein is a homologue of *Drosophila enhancer of zeste* and recruits the eed1 *polycomb*-type repressor. A region of the Ezh2 protein comprising amino acids 1-193 can interact with eed1 and repress transcription; accordingly Ezh2 and/or this functional fragment can be used as a repression domain. Denisenko et al. (1998) Mol. Cell. Biol. 18:5634-5642.

The RYBP protein is a corepressor that interacts with *polycomb* complex members and with the YY1 transcription factor. A region of RYBP comprising amino acids 42-208 has been identified as functional repression domain. Garcia et al. (1999) EMBO J. 18:3404-3418.

The RING finger protein RING1A is a member of two different vertebrate *polycomb*-type complexes, contains multiple binding sites for various components of the *polycomb* complex, and possesses transcriptional repression activity. Accordingly, RING1A or its functional fragments can serve as a repression domain. Satjin et al. (1997) Mol. Cell. Biol. 17:4105-4113.

The Bmi-1 protein is a member of a vertebrate *polycomb* complex and is involved in transcriptional silencing. It contains multiple binding sites for various *polycomb* complex components. Accordingly, Bmi-1 and its functional fragments are useful as repression domains. Gunster et al. (1997) Mol. Cell. Biol. 17:2326-2335; Hemenway et al. (1998) Oncogene 16:2541-2547.

The E2F6 protein is a member of the mammalian Bmi-1-containing *polycomb* complex and is a transcriptional repressor that is capable or recruiting RYBP, Bmi-1 and RING1A. A functional fragment of E2F6 comprising amino acids 129-281 acts as a transcriptional repression domain. Accordingly, E2F6 and its functional fragments can be used as repression domains. Trimarchi et al. (2001) Proc Natl. Acad. Sci. USA 98:1519-1524.

The eed1 protein represses transcription at least in part through recruitment of histone deacetylases *(e.g.,* HDAC2). Repression activity resides in both the N- and C-terminal regions of the protein. Accordingly, eed1 and its functional fragments can be used as repression domains. van der Vlag et al. (1999) Nature Genet. 23:474-478.

The CTBP2 protein represses transcription at least in part through recruitment of an HPC2-*polycomb* complex. Accordingly, CTBP2 and its functional fragments are useful as repression domains. Richard et al. (1999) Mol. Cell. Biol. 19:777-787.

Neuron-restrictive silencer factors are proteins that repress expression of neuron-specific genes. Accordingly, a NRSF or functional fragment thereof can serve as a repression domain. *See,* for example, US Patent No. 6,270,990.

Additional repression domains include PLZF, BCL-6, BAZF, ZNF274, PRH, TEL, TGIF, and G9A.

It will be clear to those of skill in the art that, in the formation of a fusion protein (or a nucleic acid encoding same) between a DNA-binding domain and a functional domain, either a repressor or a molecule that interacts with a repressor is suitable as a functional domain. Essentially any molecule capable of recruiting a repressive complex and/or repressive activity (such as, for example, histone deacetylation) to the target gene is useful as a repression domain of a fusion protein.

Additional exemplary activation domains include, but are not limited to, p300, CBP, PCAF, SRC1 PvALF, AtHD2A and ERF-2. See, for example, Robyr et al. (2000) Mol. Endocrinol. 14:329-347; Collingwood et al. (1999) J. Mol. Endocrinol. 23:255-275; Leo.et al. (2000) Gene 245:1-11; Manteuffel-Cymborowska (1999) Acta Biochim. Pol. 46: 77-89; McKenna et al. (1999) J. Steroid Biochem. Mol. Biol. 69:3-12; Malik et al. (2000) Trends Biochem. Sci. 25:277-283; and Lemon et al. (1999) Curr. Opin. Genet. Dev. 9:499-504. Additional exemplary activation domains include, but are not limited to, OsGAI, HALF-1, C1, AP1, ARF-5, -6, -7, and-8, CPRF1, CPRF4, MYC_RP/GP, and TRAB1. See, for example, Ogawa et al. (2000) Gene 245:21-29; Okanami et al. (1996) Genes Cells 1:87-99; Goff et al. (1991) Genes Dev. 5:298-309; Cho et al. (1999) Plant Mol. Biol. 40:419-429; Ulmason et al. (1999) Proc. Natl. Acad. Sci. USA 96: 5844-5849; Sprenger-Haussels et al. (2000) Plant J. 22:1-8; Gong et al. (1999) Plant Mol. Biol. 41:33-44; and Hobo et al. (1999) Proc. Natl. Acad. Sci. USA 96:15,348-15,353.

Additional transcriptional activation domains can be obtained from the following proteins: ATF2, myc, GATA-1, GATA-3, NF-E2, Oct1, CTF1, Sp1, GR-AF1, *a zeste* deletion, HSF-1, p53, myoD, and CARβ.

It will be clear to those of skill in the art that, in the formation of a fusion protein (or a nucleic acid encoding same) between a DNA-binding domain and a functional domain, either an activation domain or a molecule that interacts with an activation domain is suitable as a functional domain. Essentially any molecule capable of recruiting an activating complex and/or activating activity (such as, for example, histone acetylation) to the target gene is useful as an activating domain of a fusion protein.

Insulator domains, localization domains, and chromatin remodeling proteins such as ISWI-containing domains and/or methyl binding domain proteins suitable for use as functional domains in fusion molecules are described, for example, in co-owned U.S. Patent Applications 2002/0115215 and 2003/0082552 and in co-owned WO 02/44376.

In a further embodiment, a DNA-binding domain (e.g., a zinc finger domain) is fused to a bifunctional domain (BFD). A bifunctional domain is a transcriptional regulatory domain whose activity depends upon interaction of the BFD with a second molecule. The second molecule can be any type of molecule capable of influencing the functional properties of the BFD including, but not limited to, a compound, a small molecule, a peptide, a protein, a polysaccharide or a nucleic acid. An exemplary BFD is the ligand binding domain of the estrogen receptor (ER). In the presence of estradiol, the ER ligand binding domain acts as a transcriptional activator; while, in the absence of estradiol and the presence of tamoxifen or 4-hydroxy-tamoxifen, it acts as a transcriptional repressor. Another example of a BFD is the thyroid hormone receptor (TR) ligand binding domain which, in the absence of ligand, acts as a transcriptional repressor and in the presence of thyroid hormone (T3), acts as a transcriptional activator. An additional BFD is the glucocorticoid receptor (GR) ligand binding domain. In the presence of dexamethasone, this domain acts as a transcriptional activator; while, in the presence of RU486, it acts as a transcriptional repressor. An additional exemplary BFD is the ligand binding domain of the retinoic acid receptor. In the presence of its ligand all-trans-retinoic acid, the retinoic acid receptor recruits a number of co-activator complexes and activates transcription. In the absence of ligand, the retinoic acid receptor is not capable of recruiting transcriptional co-activators. Additional BFDs are known to those of skill in the art. See, for example, US Patent Nos. 5,834,266 and 5,994,313 and PCT WO 99/10508.

Another class of functional domain derived from nuclear receptors are those whose functional activity is regulated by a non-natural ligand. These are often mutants or modified versions of naturally-occurring receptors and are sometimes referred to as "switchable" domains. For example, certain mutants of the progesterone receptor (PR) are unable to interact with their natural ligand, and are therefore incapable of being transcriptionally activated by progesterone. Certain of these mutants, however, can be activated by binding small molecules other than progesterone (one example of which is the antiprogestin mifepristone). Such non-natural but functionally competent ligands have been denoted anti-hormones. See, *e.g.,* U.S. Patents 5,364,791; 5,874,534; 5,935,934; Wang et al., (1994) Proc. Natl. Acad. Sci. USA 91: 8180-8184; Wang et al. (1997) Gene Ther. 4:432-441.

Accordingly, a fusion comprising a targeted DNA-binding domain (*e.g.*, ZFP), a functional domain, and a mutant PR ligand binding domain of this type can be used for mifepristone-dependent activation or repression of an endogenous gene of choice, by designing or selecting the DNA-binding domain such that it binds in or near the gene of choice. Such fusions can further comprise a degron domain. If the fusion contains an activation domain, mifepristone-dependent activation of gene expression is obtained; if the fusion contains a repression domain, mifepristone-dependent repression of gene expression is obtained. Additionally, polynucleotides encoding such fusion proteins are provided, as are vectors comprising such polynucleotides and cells comprising such polynucleotides and vectors. It will be clear to those of skill in the art that modified or mutant versions of receptors other than PR can also be used as switchable domains. See, for example, Tora et al. (1989) EMBO J. 8:1981-1986.

## Host cells, vectors and promoters

In the practice of the disclosed methods, an engineered zinc finger protein, as described above, is expressed in a cell and binds to a target site to regulate transcription. A protein can be expressed in a cell, e.g., by delivering the protein to the cell or by delivering a polynucleotide encoding the protein to a cell. If a DNA molecule encoding the protein is delivered to a cell, it is transcribed into a mRNA molecule which can be translated to produce the protein. Alternatively, a RNA molecule can be delivered to a cell and is translated to generate the protein. Methods for polynucleotide and polypeptide delivery to cells are known in the art and exemplary methods are presented elsewhere in this disclosure. Expression of a protein in a cell can also be accomplished by transfecting a cell with a nucleic acid encoding the protein, and selecting a cell line in which the nucleic acid has stably integrated into a chromosome or is otherwise stably and heritably maintained in the cell. In these cases, expression of the protein encoded by the stably-maintained sequences can be inducible (*e.g.*, tetracycline- and doxycycline-regulated systems) or constitutive.

The disclosed compositions and methods can be used for the transcriptional regulation of any nucleotide sequence in any cell, including cultured cells, primary cells, cells in an organism and cells removed from an organism which are then returned to the organism after delivery of a protein or a polynucleotide encoding the protein to a cell. In this regard, transcription of both endogenous and exogenous sequences can be regulated. To regulate transcription of endogenous sequences, one or more copies of SEQ ID NO:1 can be inserted into or in the vicinity of the endogenous sequence to be regulated. Exemplary methods for targeted insertion of exogenous sequences into cellular genomes are disclosed in U.S. Patent Application Publication No. 2003/0232410 (Dec. 18, 2003), PCT Publication WO 03/87341 (Oct. 23, 2003) and co-owned U.S. Patent Application Serial No. 10/912,932, filed on August 6, 2004, the disclosures of which are hereby incorporated by reference in their entireties for all purposes.

As noted, the disclosed methods and compositions can also be used to regulate transcription of exogenous sequences (e.g., a vector comprising a cDNA sequence which is introduced into a cell). For example, a cDNA can be cloned into a vector containing a promoter, e.g., a SR$\alpha$ promoter, such that transcription of the cDNA sequences is controlled by the promoter. In these cases, the cDNA-containing vector is introduced into a cell, and an engineered ZFP as disclosed herein (or a polynucleotide encoding an engineered ZFP as disclosed herein) is introduced into the same cell. Alternatively, the cDNA is introduced into a stable cell line containing an integrated (or otherwise stably maintained) copy of a polynucleotide encoding an engineered ZFP as disclosed herein. In another alternative, an engineered ZFP or a polynucleotide encoding an engineered ZFP is introduced into a cell containing an integrated (or otherwise stably maintained) copy of a polynucleotide sequence comprising a cDNA.

In cells containing stably integrated sequences encoding an engineered ZFP, a number of exogenous transcription units, each containing one or more target sites for the ZFP, can be introduced (e.g., by transfection) to obtain coordinate regulation of the exogenous transcription units. Also in such cells, one or more target sites can be introduced upstream, within or adjacent to any number of endogenous transcription units, to obtain coordinate regulation of multiple genes.

Suitable vectors for the propagation of nucleotide sequences that are exogenous to a cell, and promoters for regulation of the transcription of such exogenous nucleotide sequences, are known in the art. Exemplary promoters include the SRα and CMV promoters. Regulation of transcription of sequences operatively linked to a SRα promoter can be accomplished directly using the disclosed proteins, since a copy of the target site SEQ ID NO:1 is present in the SRα promoter. Higher levels of transcription (*e.g.*, for overexpression of proteins) are achieved by placing additional copies of SEQ ID NO:1 into the SRα promoter. Furthermore, modification of a promoter other than SRα (*e.g.*, the CMV promoter), by insertion of one or more copies of SEQ ID NO:1, leads to expression levels that are higher than those obtained with the unmodified promoter.

The disclosed methods and compositions can be used in any type of cell including, but not limited to, prokaryotic cells, fungal cells, Archaeal cells, plant cells, insect cells, animal cells, vertebrate cells, mammalian cells and human cells. Suitable cell lines for protein expression are known to those of skill in the art and include, but are not limited to COS, CHO (*e.g.*, CHO-S, CHO-K1, CHO-DG44, CHO-DUXB11), VERO, MDCK, WI38, V79, B14AF28-G3, BHK, HaK, NS0, SP2/0-Agl4, HeLa, HEK293 (*e.g.*, HEK293-F, HEK293-H, HEK293-T), perC6, insect cells such as *Spodoptera fugiperda* (Sf), and fungal cells such as *Saccharomyces, Pischia* and *Schizosaccharomyces.* Progeny, variants and derivatives of these cell lines can also be used.

Exemplary promoters include SRα, CMV, phosphoglycerate kinase (PGK), human ubiquitin C (UBC), elongation factorlα (EF-lα), herpes thymidine kinase (TK), SV40 early and late promoters, human keratin-14 (K14) and Rous Sarcoma virus (RSV).

In certain embodiments, two or more promoters can be regulated with the same engineered ZFP, provided that one or more target sites for the ZFP is present in or near each promoter. This is particularly useful for recombinant production of antibodies, as sequences encoding a heavy chain can be placed under the transcriptional control of a first promoter and sequences encoding a light chain can be placed under the transcriptional control of a second promoter. The two promoters can be the same, or they can be different promoters. In additional embodiments, two copies of the same promoter, each containing a different number of ZFP target sites, can be used to obtain differential expression of sequences operatively linked to each promoter. Two different promoters, each containing a different number of target sites, can also be used.

It will be clear to those of skill in the art that it is possible to design a ZFP to bind to any predetermined target sequence; and therefore is possible to use one or more copies of such a sequence, along with a ZFP the binds to the sequence, to regulate transcription for *e.g.* overexpression.

### Fusion nucleic acids and expression vectors

In certain embodiments, a fusion polypeptide is encoded by a fusion nucleic acid. In such cases, the nucleic acid can be cloned into intermediate vectors for transformation into prokaryotic or eukaryotic cells for replication and/or expression. Intermediate vectors for storage or manipulation of the fusion nucleic acid or production of fusion protein can be prokaryotic vectors, (*e.g.*, plasmids), shuttle vectors, insect vectors, or viral vectors for example. A fusion nucleic acid can also cloned into an expression vector, for administration to a bacterial cell, fungal cell, protozoal cell, plant cell, or animal cell, preferably a mammalian cell, more preferably a human cell.

A nucleic acid encoding a fusion protein can be cloned into a vector for transformation into prokaryotic or eukaryotic cells for replication and/or expression. Vectors can be prokaryotic vectors, e.g., plasmids, or shuttle vectors, insect vectors, or eukaryotic vectors. A nucleic acid encoding a ZFP can also be cloned into an expression vector, for administration to a plant cell, animal cell, preferably a mammalian cell or a human cell, fungal cell, bacterial cell, or protozoal cell.

To obtain expression of a cloned gene or nucleic acid, sequences encoding a fusion protein are typically subcloned into an expression vector that contains a promoter to direct transcription. Suitable bacterial and eukaryotic promoters are well known in the art and described, e.g., in Sambrook et al., Molecular Cloning A Laboratory Manual (2nd ed. 1989; 3rd ed., 2001); Kriegler, Gene Transfer and Expression: A Laboratory Manual (1990); and Current Protocols in Molecular Biology (Ausubel *et al., supra.* Bacterial expression systems for expressing the ZFP are available in, e.g., *E. coli, Bacillus sp., and Salmonella* (Palva et al., Gene 22:229-235 (1983)). Kits for such expression systems are commercially available. Eukaryotic expression systems for mammalian cells, yeast, and insect cells are well known by those of skill in the art and are also commercially available.

The promoter used to direct expression of a protein-encoding nucleic acid depends on the particular application.

For example, a strong constitutive promoter is typically used for expression and purification of protein. In contrast, when a protein is administered *in vivo* for gene regulation, either a constitutive or an inducible promoter is used, depending on the particular use of the protein. In addition, a preferred promoter for administration of a protein can be a weak promoter, such as HSV TK or a promoter having similar activity. The promoter typically can also include elements that are responsive to transactivation, e.g., hypoxia response elements, Gal4 response elements, lac repressor response element, and small molecule control systems such as tet-regulated systems and the RU-486 system (*see, e.g.,* Gossen & Bujard, PNAS 89:5547 (1992); Oligino et al., Gene Ther. 5:491-496 (1998); Wang et al., Gene Ther. 4:432-441 (1997); Neering et al., Blood 88:1147-1155 (1996); and Rendahl et al., Nat. Biotechnol. 16:757-761 (1998)).

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, *e.g.,* to a nucleic acid sequence encoding the ZFP, and signals required, *e.g.,* for efficient polyadenylation of the transcript, transcriptional termination, ribosome binding sites, or translation termination. Additional elements of the cassette may include, e.g., enhancers, and heterologous splicing signals.

The particular expression vector used to transport the genetic information into the cell is selected with regard to the intended use of the protein, *e.g.*, expression in plants, animals, bacteria, fungus, protozoa, *etc.* (see expression vectors described below). Standard bacterial expression vectors include plasmids such as pBR322-based plasmids, pSKF, pET23D, and commercially available fusion expression systems such as GST and LacZ. An exemplary fusion protein is the maltose binding protein, "MBP." Such fusion proteins facilitate purification of the protein. Epitope tags, *e.g.,* c-*myc,* hemagglutinin (HA) or FLAG, can also be added to recombinant proteins to provide convenient methods of isolation, for monitoring expression, and for monitoring cellular and subcellular localization.

Expression vectors containing regulatory elements from eukaryotic viruses are often used in eukaryotic expression vectors, e.g., SV40 vectors, papilloma virus vectors, and vectors derived from Epstein-Barr virus. Other exemplary eukaryotic vectors include pMSG, pAV009/A+, pMTO10/A+ pMAMneo-5, baculovirus pDSVE, and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells.

Some expression systems have markers for selection of stably transfected cell lines such as thymidine kinase, hygromycin B phosphotransferase, and dihydrofolate reductase. High yield expression systems are also suitable, such as using a baculovirus vector in insect cells, with a protein coding sequence under the direction of the polyhedrin promoter or other strong baculovirus promoters.

The elements that are typically included in expression vectors also include a replicon that functions in E. *coli* or other prokaryotic bacteria, a gene encoding antibiotic resistance to permit selection of bacteria that harbor recombinant plasmids, and unique restriction sites in nonessential regions of the plasmid to allow insertion of recombinant sequences.

Standard transfection methods are used to produce bacterial, mammalian, yeast or insect cell lines that express large quantities of protein, which are then purified using standard techniques *(see, e.g.,* Colley et al., J. Biol. Chem. 264: 17619-17622 (1989); Guide to Protein Purification, in Methods in Enzymology, vol. 182 (Deutscher, ed., 1990)). Transformation of eukaryotic and prokaryotic cells are performed according to standard techniques (*see, e.g.,* Morrison, J. Bact. 132:349-351 (1977); Clark-Curtiss & Curtiss, Methods in Enzymology 101:347-362 (Wu et al., eds, 1983).

Any of the well known procedures for introducing foreign nucleotide sequences into host cells may be used. These include the use of calcium phosphate transfection, polybrene, protoplast fusion, electroporation, liposomes, microinjection, naked DNA, plasmid vectors, viral vectors, both episomal and integrative, and any of the other well known methods for introducing cloned genomic DNA, cDNA, synthetic DNA or other foreign genetic material into a host cell *(see, e.g.,* Sambrook *et al., supra).* It is only necessary that the particular genetic engineering procedure used be capable of successfully introducing at least one gene into the host cell capable of expressing the protein of choice.

### Nucleic acids encoding fusion proteins and delivery to cells

Conventional viral and non-viral based gene transfer methods can be used to introduce nucleic acids encoding fusion proteins and/or engineered ZFPs in cells *(e.g.,* mammalian cells) and target tissues. Such methods can also be used to administer nucleic acids encoding fusion proteins to cells *in vitro.* In certain embodiments, nucleic acids encoding fusion protiens are administered *for in vivo* or *ex vivo* gene therapy uses. Non-viral vector delivery systems include DNA plasmids, naked nucleic acid, and nucleic acid complexed with a delivery vehicle such as a liposome or poloxamer. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al.,

in Current Topics in Microbiology and Immunology Doerfler and Böhm (eds) (1995); and Yu et al., Gene Therapy 1: 13-26 (1994).

Methods of non-viral delivery of nucleic acids encoding engineered ZFPs include electroporation, lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., US 5,049,386, US 4,946,787; and US 4,897,355) and lipofection reagents are sold commercially (*e.g.,* Transfectam™, Lipofectin™, Lipofectamine®). Cationic and neutral lipids that are suitable for efficient receptor-recognition lipofection of polynucleotides include those of Felgner, WO 91/17424, WO 91/16024. Delivery can be to cells (*ex vivo* administration) or target tissues (*in vivo* administration).

The preparation of lipid:nucleic acid complexes, including targeted liposomes such as immunolipid complexes, is well known to one of skill in the art (*see, e.g.,* Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use ofRNA or DNA viral based systems for the delivery of nucleic acids encoding fusion proteins and/or engineered ZFPs take advantage of highly evolved processes for targeting a virus to specific cells in the body and trafficking the viral payload to the nucleus. Viral vectors can be administered directly to patients *(in vivo)* or they can be used to treat cells *in vitro* and the modified cells are administered to patients *(ex vivo).* Conventional viral based systems for the delivery of ZFPs include, but are not limited to, retroviral, lentivirus, adenoviral, adeno-associated, vaccinia and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof *(see, e.g.,* Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications in which transient expression of a fusion protein is preferred, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, e.g., in the *in vitro* production of nucleic acids and peptides, and for *in vivo* and *ex vivo* gene therapy procedures (*see, e.g.,* West et al., Virology 160:38-47 (1987); U.S. PatentNo. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5: 3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-102 (1995); Malech et al., PNAS 94:22 12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:91171702-3 (1998), Kearns et al., Gene Ther. 9:748-55 (1996)).

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in *trans.* Ad vectors can transduce multiple types of tissues *in vivo,* including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9: 7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and $\psi$2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in *trans* by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely *rep* and *cap,* but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Alternatively, adenovirus helper functions can be provided on a plasmid.

In many gene therapy applications, it is desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., Proc. Natl. Acad. Sci. USA 92:9747-9751 (1995), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (e.g., Fab or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Gene therapy vectors can be delivered *in vivo* by administration to an individual patient, typically by systemic administration (e.g., intravenous, intraperitoneal, intramuscular, subdermal, or intracranial infusion) or topical application, as described below. Alternatively, vectors can be delivered to cells ex *vivo,* such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector.

*Ex vivo* cell transfection for diagnostics, research, or for gene therapy (e.g., via re-infusion of the transfected cells into the host organism) is well known to those of skill in the art. In certain embodiments, cells are isolated from the subject organism, transfected with a nucleic acid (gene or cDNA), and re-infused back into the subject organism (e.g., patient). Various cell types suitable for *ex vivo* transfection are well known to those of skill in the art *(see, e.g.,* Freshney et al., Culture of Animal Cells, A Manual of Basic Technique (3rd ed. 1994)) and the references cited therein for a discussion of how to isolate and culture cells from patients).

In one embodiment, stem cells are used in *ex vivo* procedures for cell transfection and gene therapy. The advantage to using stem cells is that they can be differentiated into other cell types *in vitro,* or can be introduced into a mammal (such as the donor of the cells) where they will engraft in the bone marrow. Methods for differentiating CD34+ cells *in vitro* into clinically important immune cell types using cytokines such a GM-CSF, IFN-$\gamma$ and TNF-$\alpha$ are known (*see* Inaba et al., J. Exp. Med. 176:1693-1702 (1992)).

Stem cells are isolated for transduction and differentiation using known methods. For example, stem cells are isolated from bone marrow cells by panning the bone marrow cells with antibodies which bind unwanted cells, such as CD4+ and CD8+ (T cells), CD45+ (panB cells), GR-1 (granulocytes), and Iad (differentiated antigen presenting cells) (*see* Inaba et al., J. Exp. Med. 176:1693-1702 (1992)),

Vectors (e.g., retroviruses, adenoviruses, liposomes, etc.) containing therapeutic nucleic acids can also be administered directly to an organism for transduction of cells *in vivo.* Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions available, as described below (*see, e.g.,* Remington's Pharmaceutical Sciences, 17th ed., 1989).

DNA constructs may be introduced into the genome of a desired plant host by a variety of conventional techniques. For reviews of such techniques see, for example, Weissbach & Weissbach Methods for Plant Molecular Biology (1988, Academic Press, N.Y.) Section VIII, pp. 421-463; and Grierson & Corey, Plant Molecular Biology (1988, 2d Ed.), Blackie, London, Ch. 7-9. For example, the DNA construct may be introduced directly into the genomic DNA of the plant cell using techniques such as electroporation and microinjection of plant cell protoplasts, or the DNA constructs can be introduced directly to plant tissue using biolistic methods, such as DNA particle bombardment (see, e.g., Klein et al (1987) Nature 327:70-73). Alternatively, the DNA constructs may be combined with suitable T-DNA flanking regions and introduced into a conventional *Agrobacterium tumefaciens* host vector. *Agrobacterium tumefaciens*-mediated transformation techniques, including disarming and use of binary vectors, are well described in the scientific literature. See, for example Horsch et al (1984) Science 233:496-498, and Fraley et al (1983) Proc. Nat'l. Acad. Sci. USA 80:4803. The virulence functions of the *Agrobacterium tumefaciens* host will direct the insertion of the construct and adjacent marker into the plant cell DNA when the cell is infected by the bacteria using binary T DNA vector (Bevan (1984) Nuc. Acid Res. 12:8711-8721) or the co-cultivation procedure (Horsch et al (1985) Science 227:1229-1231). Generally, the *Agrobacterium* transformation system is used to engineer dicotyledonous plants (Bevan et al (1982) Ann. Rev. Genet 16:357-384; Rogers et al (1986) Methods Enzymol. 118:627-641). The *Agrobacterium* transformation system may also be used to transform, as well as transfer, DNA to monocotyledonous plants and plant cells. *See* Hernalsteen et al (1984) EMBO J 3:3039-3041; Hooykass-Van Slogteren et al (1984) Nature 311:763-764; Grimsley et al (1987) Nature 325:1677-179; Boulton et al (1989) Plant Mol. Biol. 12:31-40.; and Gould et al (1991) Plant Physiol. 95:426-434.

Alternative gene transfer and transformation methods include, but are not limited to, protoplast transformation through calcium-, polyethylene glycol (PEG)- or electroporation-mediated uptake of naked DNA (see Paszkowski et al. (1984) EMBO J 3:2717-2722, Potrykus et al. (1985) Molec. Gen. Genet. 199:169-177; Fromm et al. (1985) Proc. Nat. Acad. Sci. USA 82:5824-5828; and Shimamoto (1989) Nature 338:274-276) and electroporation of plant tissues (D'Halluin et al. (1992) Plant Cell 4:1495-1505). Additional methods for plant cell transformation include microinjection, silicon carbide mediated DNA uptake (Kaeppler et al. (1990) Plant Cell Reporter 9:415-418), and microprojectile bombardment (see Klein et al. (1988) Proc. Nat. Acad. Sci. USA 85:4305-4309; and Gordon-Kamm et al. (1990) Plant Cell 2:603-618).

Transformed plant cells which are produced by any of the above transformation techniques can be cultured to regenerate a whole plant which possesses the transformed genotype and thus the desired phenotype. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans, et al., "Protoplasts Isolation and Culture" in Handbook of Plant Cell Culture, pp. 124-176, Macmillian Publishing Company, New York, 1983; and Binding, Regeneration of Plants, Plant Protoplasts, pp. 21-73, CRC Press, Boca Raton, 1985. Regeneration can also be obtained from plant callus, explants, organs, pollens, embryos or parts thereof. Such regeneration techniques are described generally in Klee et al (1987) Ann. Rev. of Plant Phys. 38:467-486.

Nucleic acids introduced into a plant cell can be used to confer desired traits on essentially any plant. A wide variety of plants and plant cell systems may be engineered for the desired physiological and agronomic characteristics described herein using the nucleic acid constructs of the present disclosure and the various transformation methods mentioned above. In certain embodiments, target plants and plant cells for engineering include, but are not limited to, those monocotyledonous and dicotyledonous plants, such as crops including grain crops (e.g., wheat, maize, rice, millet, barley), fruit crops (e.g., tomato, apple, pear, strawberry, orange), forage crops (e.g., alfalfa), root vegetable crops (e.g., carrot, potato, sugar beets, yam), leafy vegetable crops (e.g., lettuce, spinach); flowering plants (e.g., petunia, rose, chrysanthemum), conifers and pine trees (e.g., pine fir, spruce); plants used in phytoremediation (e.g., heavy metal accumulating plants); oil crops (e.g., sunflower, rape seed) and plants used for experimental purposes (e.g., *Arabidopsis*). Thus, the disclosed methods and compositions have use over a broad range of plants, including, but not limited to, species from the genera Asparagus, Avena, Brassica, Citrus, Citrullus, Capsicum, Cucurbita, Daucus, Glycine, Hordeum, Lactuca, Lycopersicon, Malus, Manihot, Nicotiana, Oryza, Persea, Pisum, Pyrus, Prunus, Raphanus, Secale, Solanum, Sorghum, Triticum, Vitis, Vigna, and Zea.

One of skill in the art will recognize that after the expression cassette is stably incorporated in transgenic plants and confirmed to be operable, it can be introduced into other plants by sexual crossing. Any of a number of standard breeding techniques can be used, depending upon the species to be crossed.

A transformed plant cell, callus, tissue or plant may be identified and isolated by selecting or screening the engineered plant material for traits encoded by the marker genes present on the transforming DNA. For instance, selection may be performed by growing the engineered plant material on media containing an inhibitory amount of the antibiotic or herbicide to which the transforming gene construct confers resistance. Further, transformed plants and plant cells may also be identified by screening for the activities of any visible marker genes (e.g., the β-glucuronidase, luciferase, B or C1 genes) that may be present on the recombinant nucleic acid constructs. Such selection and screening methodologies are well known to those skilled in the art.

Physical and biochemical methods also may be used to identify plant or plant cell transformants containing inserted gene constructs. These methods include but are not limited to: 1) Southern analysis or PCR amplification for detecting and determining the structure of the recombinant DNA insert; 2) Northern blot, S1 RNase protection, primer-extension or reverse transcriptase-PCR amplification for detecting and examining RNA transcripts of the gene constructs; 3) enzymatic assays for detecting enzyme or ribozyme activity, where such gene products are encoded by the gene construct; 4) protein gel electrophoresis, Western blot techniques, immunoprecipitation, or enzyme-linked immunoassays, where the gene construct products are proteins. Additional techniques, such *as in situ* hybridization, enzyme staining, and immunostaining, also may be used to detect the presence or expression of the recombinant construct in specific plant organs and tissues. The methods for doing all these assays are well known to those skilled in the art.

Effects of gene manipulation using the methods disclosed herein can be observed by, for example, northern blots of the RNA (*e.g.,* mRNA) isolated from the tissues of interest. Typically, if the amount of mRNA has increased, it can be assumed that the corresponding endogenous gene is being expressed at a greater rate than before. Other methods of measuring gene activity can be used. Different types of enzymatic assays can be used, depending on the substrate used and the method of detecting the increase or decrease of a reaction product or by-product. In addition, the levels of protein expressed can be measured immunochemically, i.e., ELISA, RIA, EIA and other antibody based assays well known to those of skill in the art, such as by electrophoretic detection assays (either with staining or western blotting). The transgene may be selectively expressed in some tissues of the plant or at some developmental stages, or the transgene may be expressed in substantially all plant tissues, substantially along its entire life cycle. However, any combinatorial expression mode is also applicable.

The present disclosure also encompasses seeds of the transgenic plants described above wherein the seed has the transgene or gene construct. The present disclosure further encompasses the progeny, clones, cell lines or cells of the transgenic plants described above wherein said progeny, clone, cell line or cell has the transgene or gene construct.

**Delivery vehicles**

An important factor in the administration of polypeptide compounds, such as fusion proteins, is ensuring that the polypeptide has the ability to traverse the plasma membrane of a cell, or the membrane of an intra-cellular compartment such as the nucleus. Cellular membranes are composed of lipid-protein bilayers that are freely permeable to small, nonionic lipophilic compounds and are inherently impermeable to polar compounds, macromolecules, and therapeutic or diagnostic agents. However, proteins and other compounds such as liposomes have been described, which have the ability to translocate polypeptides across a cell membrane.

For example, "membrane translocation polypeptides" have amphiphilic or hydrophobic amino acid subsequences that have the ability to act as membrane-translocating carriers. In one embodiment, homeodomain proteins have the ability to translocate across cell membranes. The shortest internalizable peptide of a homeodomain protein, Antennapedia, was found to be the third helix of the protein, from amino acid position 43 to 58 (*see, e.g.,* Prochiantz, Current Opinion in Neurobiology 6:629-634 (1996)). Another subsequence, the h (hydrophobic) domain of signal peptides, was found to have similar cell membrane translocation characteristics (*see, e.g.,* Lin et al., J. Biol. Chem. 270:1 4253-14258 (1995)).

Examples of peptide sequences which can be linked to a protein, for facilitating uptake of the protein into cells, include, but are not limited to: an 11 amino acid peptide of the tat protein of HIV; a 20 residue peptide sequence which corresponds to amino acids 84-103 of the p16 protein (*see* Fahraeus et al., Current Biology 6:84 (1996)); the third helix of the 60-amino acid long homeodomain of Antennapedia (Derossi et al., J. Biol. Chem. 269:10444 (1994)); the h region of a signal peptide such as the Kaposi fibroblast growth factor (K-FGF) h region (Lin *et al., supra);* or the VP22 translocation domain from HSV (Elliot & O'Hare, Cell 88:223-233 (1997)). Other suitable chemical moieties that provide enhanced cellular uptake may also be chemically linked to ZFPs. Membrane translocation domains (*i,e.,* internalization domains) can also be selected from libraries of randomized peptide sequences. *See,* for example, Yeh et al. (2003) Molecular Therapy 7(5):S461, Abstract #1191.

Toxin molecules also have the ability to transport polypeptides across cell membranes. Often, such molecules (called "binary toxins") are composed of at least two parts: a translocation/binding domain or polypeptide and a separate toxin domain or polypeptide. Typically, the translocation domain or polypeptide binds to a cellular receptor, and then the toxin is transported into the cell. Several bacterial toxins, including *Clostridium perfringens* iota toxin, diphtheria toxin (DT), *Pseudomonas* exotoxin A (PE), pertussis toxin (PT), *Bacillus anthracis* toxin, and pertussis adenylate cyclase (CYA), have been used to deliver peptides to the cell cytosol as internal or amino-terminal fusions (Arora et al., J. Biol. Chem., 268:3334-3341 (1993); Perelle et al., Infect. Immun., 61:5147-5156 (1993); Stenmark et al., J. Cell Biol. 113:

1025-1032 (1991); Donnelly et al., PNAS 90:3530-3534 (1993); Carbonetti et al., Abstr. Annu. Meet. Am. Soc. Microbiol. 95:295 (1995); Sebo et al., Infect. Immun. 63:3851-3857 (1995); Klimpel et al., PNAS U.S.A. 89:10277-10281 (1992); and Novak et al., J. Biol. Chem. 267:17186-17193 1992)).

Such peptide sequences can be used to translocate ZFPs and other types of fusion proteins across a cell membrane. Polypeptide sequences can be conveniently fused to or derivatized with such translocation sequences. Typically, the translocation sequence is provided as part of a fusion protein. Optionally, a linker can be used to link the translocation sequence to the remainder of the fusion protein. Any suitable linker can be used, e.g., a peptide linker. *See supra.*

A fusion protein can also be introduced into an animal cell, preferably a mammalian cell, via a liposomes and liposome derivatives such as immunoliposomes. The term "liposome" refers to vesicles comprised of one or more concentrically ordered lipid bilayers, which encapsulate an aqueous phase. The aqueous phase typically contains the compound to be delivered to the cell, *e.g.* a fusion protein comprising a degron domain and a ZFP DNA-binding domain.

The liposome fuses with the plasma membrane, thereby releasing the protein into the cytosol. Alternatively, the liposome is phagocytosed or taken up by the cell in a transport vesicle. Once in the endosome or phagosome, the liposome either degrades or fuses with the membrane of the transport vesicle and releases its contents.

In current methods of drug delivery via liposomes, the liposome ultimately becomes permeable and releases the encapsulated compound (in this case, a fusion protein) at the target tissue or cell. For systemic or tissue specific delivery, this can be accomplished, for example, in a passive manner wherein the liposome bilayer degrades over time through the action of various agents in the body. Alternatively, active drug release involves using an agent to induce a permeability change in the liposome vesicle. Liposome membranes can be constructed so that they become destabilized when the environment becomes acidic near the liposome membrane *(see, e.g.,* PNAS 84:7851 (1987); Biochemistry 28:908 (1989)). When liposomes are endocytosed by a target cell, for example, they become destabilized and release their contents. This destabilization is termed fusogenesis.

Dioleoylphosphatidylethanolamine (DOPE) is the basis of many "fusogenic" systems.

Such liposomes typically comprise a protein and a lipid component, e.g., a neutral and/or cationic lipid, optionally including a receptor-recognition molecule such as an antibody that binds to a predetermined cell surface receptor or ligand (e.g., an antigen). A variety of methods are available for preparing liposomes as described in, *e.g.*, Szoka et al., Ann. Rev. Biophys. Bioeng. 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,501,728, 4,774,085, 4,837,028, 4,946,787, PCT Publication No. WO 91/17424, Deamer & Bangham, Biochim. Biophys. Acta 443:629-634 (1976); Fraley, et al., PNAS 76:3348-3352 (1979); Hope et al., Biochim. Biophys. Acta 812:55-6S (1985); Mayer et al., Biochim. Biophys. Acta 858: 161-168 (1986); Williams et al., PNAS 85:242-246 (1988); Liposomes (Ostro (ed.), 1983, Chapter 1); Hope et al., Chem. Phys. Lip. 40:89 (1986); Gregoriadis, Liposome Technology (1984) and Lasic, Liposomes: from Physics to Applications (1993)). Suitable methods include, for example, sonication, extrusion, high pressure/homogenization, microfluidization, detergent dialysis, calcium-induced fusion of small liposome vesicles and ether-fusion methods, all of which are known to those of skill in the art.

In certain embodiments, it is desirable to target liposomes using targeting moieties that are specific to a particular cell type, tissue, and the like. Targeting of liposomes using a variety of targeting moieties (e.g., ligands, receptors, and monoclonal antibodies) has been described. *See, e.g.,* U.S. Patent Nos. 4,957,773 and 4,603,044.

Examples of targeting moieties include monoclonal antibodies specific to antigens associated with neoplasms, such as prostate cancer specific antigen and MAGE. Tumors can also be targeted by detecting gene products resulting from the activation or over-expression of oncogenes, such as ras or c-erbB2. In addition, many tumors express antigens normally expressed by fetal tissue, such as the alphafetoprotein (AFP) and carcinoembryonic antigen (CEA). Sites of viral infection can be targeted using various viral antigens such as hepatitis B core and surface antigens (HBVc, HBVs) hepatitis C antigens, Epstein-Barr virus antigens, human immunodeficiency type-1 virus (HIV1) and papilloma virus antigens. Inflammation can be detected using molecules specifically recognized by surface molecules which are expressed at sites of inflammation such as integrins (e.g., VCAM-1), selectin receptors (*e.g.,* ELAM-1) and the like.

Standard methods for coupling targeting agents to liposomes can be used. These methods generally involve incorporation into liposomes of lipid components, e.g., phosphatidylethanolamine, which can be activated for attachment of targeting agents, or derivatized lipophilic compounds, such as lipid derivatized bleomycin. Antibody targeted liposomes can be constructed using, for instance, liposomes which incorporate protein A (*see* Renneisen et al., J. Biol. Chem., 265:16337-16342 (1990) and Leonetti et al., PNAS 87:2448-2451 (1990).

### Dosages

For therapeutic applications, the dose administered to a patient, in the context of the present disclosure, should be sufficient to effect a beneficial therapeutic response in the patient over time. In addition, particular dosage regimens can be useful for determining phenotypic changes in an experimental setting, *e.g.,* in functional genomics studies, and in cell or animal models. The dose will be determined by the efficacy and $K_d$ of the particular ZFP employed, the nuclear volume of the target cell, and the condition of the patient, as well as the body weight or surface area of the patient to be treated. The size of the dose also will be determined by the existence, nature, and extent of any adverse side-effects that accompany the administration of a particular compound or vector in a particular patient.

As an example, the maximum therapeutically effective dosage of ZFP for approximately 99% binding to target sites is calculated to be in the range of less than about $1.5x10^5$ to $1.5x10^6$ copies of the specific ZFP molecule per cell. The number of ZFPs per cell for this level of binding is calculated as follows, using the volume of a HeLa cell nucleus (approximately 1000 $\mu m^3$ or $10^{-12}$ L; *Cell Biology,* (Altman & Katz, eds. (1976)). As the HeLa nucleus is relatively large, this dosage number is recalculated as needed using the volume of the target cell nucleus. This calculation also does not take into account competition for ZFP binding by other sites. This calculation also assumes that essentially all of the ZFP is localized to the nucleus. A value of 100x $K_d$ is used to calculate approximately 99% binding of to the target site, and a value of $10\times K_d$ is used to calculate approximately 90% binding of to the target site. For this example, $K_d$ = 25 nM

ZFP + target site $\leftrightarrow$ complex
i.e., DNA + protein $\leftrightarrow$DNA:protein complex

$$K_d = \frac{[DNA]\,[protein]}{[DNA:protein\ complex]}$$

When 50% of ZFP is bound, $K_d$ = [protein]
So when [protein] = 25 nM and the nucleus volume is $10^{-12}$ L
[protein] = $(25\times10^{-9}$ moles/L) ($10^{-12}$ L/nucleus) ($6\times10^{23}$ molecules/mole)
=15,000 molecules/nucleus for 50% binding When 99% target is bound; $100\times K_d$ = [protein]
$100\times K_d$ = [protein] = 2.5 $\mu$M
($2.5\times10^{-6}$moles/L) ($10^{-12}$L/nucleus) ($6\times10^{23}$ molocules/mole)
= about 1,500,000 molecules per nucleus for 99% binding of target site.

The appropriate dose of an expression vector encoding a ZFP fusion protein can also be calculated by taking into account the average rate of ZFP expression from the promoter and the average rate of ZFP degradation in the cell. In certain embodiments, a weak promoter such as a wild-type or mutant HSV TK promoter is used, as described above. The dose of fusion protein in micrograms is calculated by taking into account the molecular weight of the particular protein being employed.

In determining the effective amount of the protein to be administered in the treatment or prophylaxis of disease, the physician evaluates circulating plasma levels of the protein or nucleic acid encoding the protein, potential toxicities due to the protein, progression of the disease, and the production of antibodies to the protein. Administration can be accomplished via single or divided doses.

Pharmaceutical compositions and administration

Fusion proteins and expression vectors encoding such proteins can be administered directly to the patient for targeted gene regulation, targeted DNA cleavage and/or recombination, and for therapeutic or prophylactic applications, for example, cancer, ischemia, diabetic retinopathy, macular degeneration, rheumatoid arthritis, psoriasis, HIV infection, sickle cell anemia, Alzheimer's disease, muscular dystrophy, neurodegenerative diseases, vascular disease, cystic fibrosis, stroke, and the like. Examples of microorganisms that can be inhibited by ZFP gene therapy include pathogenic bacteria, e.g., chlamydia, rickettsial bacteria, mycobacteria, staphylococci, streptococci, pneumococci, meningococci and conococci, klebsiella, proteus, serratia, pseudomonas, legionella, diphtheria, salmonella, bacilli, cholera, tetanus, botulism, anthrax, plague, leptospirosis, and Lyme disease bacteria; infectious fungus, e.g., *Aspergillus, Candida* species; protozoa such as sporozoa (e.g., *Plasmodia),* rhizopods (e.g., *Entamoeba)* and flagellates *(Trypanosoma, Leishmania, Trichomonas, Giardia,* etc.);viral diseases, e.g., hepatitis (A, B, or C), herpes virus (*e.g.*, VZV, HSV-1, HSV-6, HSV-II, CMV, and EBV), HIV, Ebola, adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, poliovirus, rabies virus, and arboviral encephalitis virus, *etc.*

Administration of therapeutically effective amounts is by any of the routes normally used for introducing a protein or nucleic acid into ultimate contact with the tissue to be treated. The fusion proteins or encoding nucleic acids are administered in any suitable manner, preferably with pharmaceutically acceptable carriers. Suitable methods of administering such modulators are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions that are available (*see, e.g.,* Remington's Pharmaceutical Sciences, 17th ed. 1985)).

Fusion proteins or their encoding nucleic acids, alone or in combination with other suitable components, can be made into aerosol formulations (*i.e.*, they can be "nebulized") to be administered via inhalation. Aerosol formulations can be placed into pressurized acceptable propellants, such as dichlorodifluoromethane, propane, nitrogen, and the like.

Formulations suitable for parenteral administration, such as, for example, by intravenous, intramuscular, intradermal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The disclosed compositions can be administered, for example, by intravenous infusion, orally, topically, intraperitoneally, intravesically or intrathecally. The formulations of compounds can be presented in unit-dose or multi-dose sealed containers, such as ampules and vials. Injection solutions and suspensions can be prepared from sterile powders, granules, and tablets of the kind previously described.

## Applications

The disclosed proteins can also be used *in vitro.* For example, the engineered ZFP (or a transcription/translation lysate) can be incubated with a polynucleotide (e.g., plasmid) e.g., in a coupled transcription/translation system for protein production *in vitro.*

In additional embodiments, the target site for an engineered ZFP can be placed on either side of a coding sequence, either endogenous or exogenous. In this situation, binding of an engineered ZFP (optionally fused to, *e.g.*, an insulator domain; *see e.g.,* WO 01/02553; WO 02/44376) to the target sites can protect the coding sequence against the influence of e.g., position effects and heterochromatinization.

The disclosed methods can be used in concert with existing methods (e.g., methotrexate-based selection for amplification of sequences adjacent to a dihydrofolate reductase gene) to obtain increased levels of protein expression based on both increased template number and higher levels of transcription.

In additional embodiments, a target sequence for an engineered ZFP can be introduced adjacent to or overlapping a binding site for another transcriptional regulatory molecule such that regulation can be further modulated by the engineered ZFP. Engineered ZFPS fused to either transcriptional activation domains or transcriptional repression domain can be used, as can ZFPs not fused to a functional domain, which can sterically modulate binding of other regulatory molecules.

## Inactivation of endogenous genes to facilitate protein production

In certain embodiments, the function of one or more cellular gene products is inactivated to enhance the level and/or quality of proteins produced using the methods and compositions disclosed herein. Gene function can be inactivated by, *e.g.,* disruption of one or more alleles of the endogenous cellular gene that encodes the gene product (e.g., by gene "knock-out") and/or by repressing transcription of an endogenous cellular gene as described, for example, in co-owned U.S. Patent No. 6,534,261.

Disruption of an endogenous cellular gene can be accomplished by mutagenesis (e.g., chemical or radiation-induced) or by insertion of exogenous DNA sequences into the cellular genome, optionally followed by selection for the desired mutant cells. Insertion of exogenous sequences can be random (e.g., following retroviral infection or contact of a cell with a DNA molecule) or targeted (e.g., U.S. Patent No. 5,614,396). Targeted insertion of exogenous sequences can also be accomplished by targeted cleavage of genomic DNA in combination with introduction of exogenous DNA. Co-owned PCT WO 2005/014791 and United States Provisional Patent Application 60/702,394 (filed July 26, 2005), the disclosures of which are incorporated by reference, provide methods and compositions for homology-dependent and homology independent methods, respectively, for targeted insertion of exogenous sequences. In addition, PCT WO 2005/014791 also discloses methods for targeted mutagenesis by targeted DNA cleavage followed by non-homologous end-joining.

By way of example, one or more genes involved in the process of apoptosis (or the function thereof) can be inactivated. Exemplary genes involved in apoptosis are shown in Table 1.

**Table 1**

| |
|---|
| caspase1 |
| caspase2 |
| caspase3 |
| caspase4 |
| caspase5 |
| caspase6 |
| caspase7 |
| caspase8 |
| caspase9 |
| caspase10 |
| caspase11 |
| caspase12 |
| caspase13 |
| caspase14 |
| bax |
| bak |
| bik |
| APAF-1 (apoptosome associated factor 1) |
| c-jun |
| lactate dehydrogenase |
| cytochrome c |

Genes whose products negatively regulate cell cycle progression can be inactivated. Exemplary genes involved in regulation of the cell cycle are shown in Table 2.

**Table 2**

| |
|---|
| p16 |
| p19 |
| Rb (retinoblastoma protein) |
| p53 |
| p73 |
| Telomerase |

Because the bioactivity of certain proteins can be affected by their glycosylation pattern, it may be desirable, in certain circumstances, to inactivate certain genes whose products are involved in protein glycosylation. Exemplary genes involved in glycosylation are shown in Table 3.

Table 3

| |
|---|
| a-1,6-fucosyltransferase |
| CMP-N-acetylneuramlnic acid hydroxylase |
| CMP-sialic acid hydroxylase |
| Glucosamine-6-phosphate lsomerase |

Genes encoding receptors for infection (*e.g.,* viral or bacterial receptors) can be inactivated to enhance protein production. For example, the gene encoding the minute virus of mice (MVM) receptor can be inactivated.

In certain cases, it may be desirable to integrate an exogenous DNA molecule encoding a transgene and a selection marker (*e.g.,* to select cells containing the integrated transgene). If the selection marker is encoded by the genome of the cell into which it is desired to integrate the exogenous DNA molecule, it can be useful to inactivate the endogenous gene encoding that selection marker. Inactivation can be partial (such that reduced levels of the marker are produced) or complete (such that no marker is produced). Exemplary marker genes that can be so inactivated are shown in Table 4.

**Table 4**

| |
|---|
| dihydrofolate reducatase |
| Glutamine synthetase |
| hypoxanthine phosphoribosyl transferase |

Genes encoding proteases can be inactivated to enhance protein production. Exemplary proteases are shown in Table 5.

**Table 5**

| |
|---|
| serine proteases |
| elastase |
| coliagenase |
| plasminogen activator |

For safety and compliance with regulatory requirements, genes encoding prions (PRPs)and/or pseudo-prions can be inactivated. Pseudo-prions include, for example, PRNP/PRP, Prion-like protein Doppel (PRND) and Shadow ofPrion (SPRN). *See,* for example, Liao et al. (1986) Science 233:364-367 (PRNP); Lu et al. (2000) Biochemistry 39: 13575-13583 (PRND) and Premzl et al. (2003) Gene 314:89-102 (SPRN).

In cells which are being used for production of antibody molecules, it can be useful to inactivate genes encoding proteins which are bound by the antibody or antibodies being produced. For example, genes encoding the antigen(s) recognized by the antibody or antibodies can be inactivated by any of the methods disclosed herein.

Additional cellular processes which can affect protein yield and quality include, for example, replication, transcription, RNA processing, translation, amino acid biosynthesis, cellular metabolism, protein folding, protein degradation, protein transport, stress responses and plasmid copy number control. It is to be understood that either positive or negative regulation of a gene (including inactivation or gene "knock-out") involved in any of the aforementioned processes can be used to enhance the yield and/or quality of a protein expressed using the methods and compositions disclosed herein.

## EXAMPLES

The following examples are presented as illustrative of, but not limiting, the claimed subject matter.

### Example 1: Construction of a SR$\alpha$ promoter

The SV40 early promoter was generated by PCR from the plasmid pRL-SV40 (Promega) and included base pairs 80-449 from the pRL-SV40 sequence and incorporated the HindIII site of the promoter at the 3'end. Bases 80-88 comprise the 9bp target site for the 2392/00 and 2932/10 ZFPs. A Bpu101restriction site was added to the PCR primer immediately upstream of the ZFP binding site to facilitate subsequent cloning into the backbone vector, pcDNA3.1/zeo, from which the original CMV promoter had been removed.

The R-U5 composite sequence was generated by PCR from the plasmid pDrive01-NSE(r)-RU5 v02 (Invivogen) and included base pairs 1774-2066 from the plasmid sequence, which encompass the HindIII restriction site within the 5' primer binding site. An EcoRI restriction site was added to the 3' PCR primer to facilitate cloning into the backbone vector.

The SV40 and RU5 sequences were linked at their respective HindIII sites to form the SV40-RU5 fusion. The sequence of this hybrid promoter (SEQ ID NO:9) is shown in Figure 1, with the ZFP target site underlined.

### Example 2: Engineering zinc finger proteins to bind to a target site in the SR$\alpha$ promoter

To increase expression of proteins whose coding regions are under the control of the SR$\alpha$ promoter, a three-finger zinc finger protein, SBS2392/00, was synthesized (according to methods disclosed in co-owned U.S. Patents 6,453,242 and 6,534,261) to bind a nine-nucleotide target sequence in the SR$\alpha$ promoter. The target site was the sequence GCTGTGGAA (SEQ ID NO:1), located as shown in Figure 1. The sequence of the protein is as follows, with the recognition regions of the zinc fingers underlined:

KKKQHICHIQGCGKVYGQRSNLVRHLRWHTGERPFMCTWSYCGKRFTRSDA LSRHKRTHTGEKKFACPECPKRFMQSSDLRRHIKTHQNK (SEQ ID NO:2).

Additional zinc finger domains capable of binding to SEQ ID NO:1 were obtained using a two-hybrid selection system. *See, e.g.,* U.S. Patent Application Publication No. 2003/0044787 (Mar. 6, 2003) and Joung et al. (2000) Proc. Natl. Acad. Sci. USA 97:7382-7287. Their amino acid sequences are shown in Figure 6.

Nucleotide sequences encoding these zinc finger proteins were fused to nucleotide sequences encoding a VP16 activation domain, a nuclear localization signal (NLS) and a FLAG epitope tag to generate engineered transcription factors. Exemplary polynucleotide sequences are shown in Figures 3 and 5, with their encoded amino acid sequences shown in Figures 2 and 4, respectively.

### Example 3: Enhancement of SRα promoter-driven expression of immunoglobulin genes by an engineered zinc finger protein

A DG44 CHO cell line containing an integrated antibody expression construct driven by the SRα promoter was transiently transfected with 20-250 ng of plasmid encoding the VP16 activation domain (NVF), or ZFP 2392/00 (SEQ ID NO:2)linked to VP16 (2392-VP16). Immunoglobulin kappa chain mRNA expression was measured by real-time PCR (Taqman®). The results, shown in Figure 7, indicate that SRα-driven transcription of kappa chain mRNA is increased 4-5-fold by the 2392/00 transcription factor.

In an experiment to measure the effect of the 2392/00-VP16 fusion on protein levels, two different DG44 CHO cell-derived cell lines (Line A and Line B), that stably express gamma heavy chains and kappa light chains, the expression of both of which is driven by the SRα promoter, were transiently transfected with plasmid encoding the VP16 activation domain (NVF) or ZFP 2392/00 linked to the VP16 activation domain (2392). Expression of secreted immunoglobulin G was measured by ELISA. The results, shown in Figure 8, indicate that, in both cell lines, expression ofIgG was increased by the 2392/00 ZFP-VP16 fusion.

To determine whether the 2392/00-VP16 fusion protein can increase expression in an optimized expression system, a cell line containing amplified gamma heavy chain and kappa light chain cDNAs, obtained by methotrexate selection, was used. Sequences encoding the 2392/00-VP16 fusion ZFP were introduced into cells of this "high producer" line (ZFP) and the level of mRNA expression was compared with the same cells in the absence of ZFP (NT). Figure 9 shows that optimized levels of both gamma chain and kappa chain mRNAs can be increased a further three-fold when the 2392/00-VP16 protein is introduced into these cells.

A cell line containing integrated cDNAs encoding the gamma heavy chain and kappa light chain components of immunoglobulin G, both under the transcriptional control of the SRα promoter, was transfected with a plasmid encoding the 2392/00-VP16 fusion protein. Transfected cells were selected for stable ZFP expression. Secreted IgG was measured by ELISA from a clonal line stably expressing the 2392/00-VP16 fusion protein. The results, shown in Figure 10, reveal an approximately 4-fold increase in secreted IgG in cells stably transfected with the 2392/00-VP16 protein, compared to control untransfected cells.

### Example 4: Selection and properties of a stable cell line containing integrated sequences encoding a 2392/10-VP16 fusion protein

Chinese hamster ovary (CHO) DG44 cells growing adherent on 6 well plates were transfected with a plasmid encoding a 2392/10-VP16 fusion protein, as follows. Cells were incubated with 1μg DNA and 4μl Lipofectamine 2000 in 1ml serum-free growth medium for 4hrs, then medium was aspirated and 2ml of regular growth medium added. After 3 days, cells were split in various dilutions into 25 cm dishes and subjected to Zeocin selection. When selection was complete, individual colonies were harvested from the appropriate dilution plate, transferred into 24 well dishes and grown up. Criteria for completion of selection were that individual cell colonies appear and no new cell death occurs, while complete cell death is observed in non-transfected DG44 cells that had been placed under selection at the same time. 22 clonal lines were analyzed for ZFP expression, cell growth properties and activation of reporter constructs containing mulitmerized ZFP binding sites.

One of these lines was subcloned and genomic DNA was extracted from two of the subclones. The DNA was digested completely with BamHI restriction endonuclease, resolved on a 0.9% TAE agarose gel, transferred to Nytran$^+$ membrane and the membrane was probed with a 290bp radiolabelled DNA fragment encoding the VP16 activation domain. A single band of approximately 12 kbp was detected in the two ZFP-containing cell lines, suggesting a single copy of the DNA encoding the fusion proteins is present. This band was not detected in DNA from wild type DG44 CHO cells.

**Example 5: Construction of a SRα promoter containing multiple copies of a target site for a ZFP-VP16 fusion protein**

A promoter denoted "SRαZ6" was constructed by joining a DNA fragment containing six copies of the target site for the 2392/00 and 2392/10 ZFPs (SEQ ID NO:1) to an SRα promoter-containing DNA fragment. The Z6 promoter thus contains 7 copies of SEQ ID NO:1. These additional target sites confer a much greater level of ZFP-mediated activation of this promoter. *See infra.*

The insert containing the additional six binding sites was inserted as a BamHI/NdeI fragment after first inserting those restriction sites (shown in bold text below) upstream of the SV40 portion of the SRα promoter. The seven iterations of the target site are underlined. The target site downstream of the NdeI site is the site originally present in the SV40 portion of the SRα promoter.

The sequence of that portion of the SRαZ6 promoter containing the ZFP target sites is as follows:

**BamHI**

**ggatcc**gagctgtggaatgagagctgtggaatgagagctgtggaatgagagc
tgtggaatgagagctgtggaatgagagctgtggaatga**catatg**gctgtggaatgtg
tgtcagtta **NdeI**

**(SEQ ID NO:23)**

**Example 6: Construction of a CMV promoter containing multiple copies of a target site for a ZFP-VP16 fusion protein**

A promoter denoted "CMVz10" was constructed by inserting a DNA fragment, comprising multiple copies of a target site (SEQ ID NO:1) for the 2392/00 and 2392/10 engineered ZFPs, into a MluI restriction site immediately upstream of the CMV promoter. The CMVz10 promoter contains 9 perfect iterations of the target site plus a single 8/9 match in the tenth iteration (a C to T substitution). The sequence of the CMVz10 promoter is shown below, with the MluI restriction sites shown in bold and the target sites underlined. Note that, since this sequence was inserted in the reverse orientation, the sequence shown reflects the complement of each binding site.

**MluI**

**acgcgt**tcattccacagctctcattccacagctctcattccacagctctcat
tccacagctctcattccacagctctcattccacagctctcattccacagctctcatt
ccacagctctcattccacagctctcattccacagt**cacgcgt**
**MluI**

**(SEQ ID NO:24)**

Additional promoter constructs, which include different numbers of binding sites and different orientations of the binding sites with respect to the promoter, have also been constructed and tested.

**Example 7: Enhanced protein production from a SRα promoter containing multiple copies of a target site for a ZFP-VP16 fusion protein**

A clonal isolate (2392/10-7) of the DG44 CHO cell line containing the stably integrated 2392/10 ZFP (Example 4), and parent DG44 cells, were transfected with two different antibody-expressing constructs. In one, heavy chain and the light chain transcription units were each under the control of an SRα promoter (SRα); in the other, both the heavy chain and light chain transcription units were under the control of an SRα promoter containing an additional 8 target sites for the ZFP immediately upstream of the promoter (SRa 2393BS). Three days after transfection, IgG secretion was measured by ELISA.

The results are shown in Figure 11, and indicate that IgG expression level is dependent upon the presence of the 2392/10 ZFP and that more copies of the 2392/10 target site result in higher IgG levels.

**Example 8: Enhanced transcription from a CMV promoter containing multiple copies of a target site for a ZFP-VP16 fusion protein**

To test the effect of the 2392/10- VP16 fusion protein on the transcriptional activity of the CMV promoter, a reporter construct, in which a CMV promoter was operatively linked to sequences encoding green fluorescent protein (GFP) was constructed. Variants of this reporter construct were then constructed, which contained multiple copies of the target site for the 2392/10 ZFP upstream of the core CMV promoter sequences. Promoters containing different numbers of target sites in different orientations were tested by transfecting them into the 2392/10-7 cell line (Example 4), followed by analysis of GFP mRNA levels by real-time PCR (Taqman®) Representative results are shown in Figure 12. The results indicate that more copies of the target site lead to higher steady-state mRNA levels, and that the effect is independent of the orientation of the target sites with respect to the remainder of the CMV promoter sequences. No effect of either number or orientation of target sites is observed in a parental cell line that does not express the 2392/10 ZFP.

**Example 9: Enhanced expression of erythropoietin, mediated by the 2392110-VP16 fusion protein, from SRα and CMV promoters containing multiple copies of the 2392/10 target site**

Three different erythropoietin (Epo)-expressing constructs were constructed. In the first, Epo expression was controlled by a CMV promoter fused to a β-globin intron (CMV). In the second, Epo expression was controlled by a CMV promoter containing ten upstream copies of the 2392/10 binding site, fused to a β-globin intron (CMVz10, Example 6). In the third, Epo expression was controlled by a SRα promoter with six additional copies of the 2392/10 binding site immediately upstream of the promoter (SRαZ6, Example 5). The three constructs were transiently transfected into either parental DG44 CHO cells or the ZFP-expressing 2392/10-7 cell line. After 24 hours, Epo secretion was measured by ELISA.

The results, shown in Figure 13, confirm previous observations that, in the absence of the 2392/10-VP16 ZFP, the CMV promoter is stronger than the SRα promoter. However, activation of the SRαZ6 promoter by the 2393/10-VP16 protein increases its activity such that it is comparable to that of the CMV promoter. Moreover, insertion of 2392/10 target sites adjacent to the CMV promoter increases its already strong activity an additional three-fold in 2392/10-VP 16-expressing cells. Thus, the activity of the strongest known naturally-occurring promoter has been improved by the use of an engineered ZFP transcriptional activator.

All patents, patent applications and publications mentioned herein are hereby incorporated by reference in their entirety.

Although disclosure has been provided in some detail by way of illustration and example for the purposes of clarity of understanding, it will be apparent to those skilled in the art that various changes and modifications can be practiced without departing from the spirit or scope of the disclosure. Accordingly, the foregoing descriptions and examples should not be construed as limiting.

The following items are part of the specification:

1. A method for regulating the transcription of a nucleotide sequence in a cell, the method comprising:

    expressing, in the cell, a protein comprising SEQ ID N0:7 wherein the protein binds to a target site;
    wherein the target site is operatively linked to the nucleotide sequence.

2. The method of item 1, wherein the target site comprises SEQ ID N0:1.

3. The method of item 2, wherein a plurality of target sites are operatively linked to the nucleotide sequence.

4. The method of item 1, wherein the nucleotide sequence comprises a cDNA sequence.

5. The method of item 1, wherein the protein further comprises a transcriptional activation domain.

6. The method of item 5, wherein the transcriptional activation domain is the VP16 domain.

7. A method for regulating the transcription of first and second nucleotide sequences in a cell, the method comprising:

expressing, in the cell, a protein comprising SEQ ID N0:7 wherein the protein binds to a target site;
wherein the target site is operatively linked to both of the first and second nucleotide sequences.

8. The method of item 7, wherein the target site comprises SEQ ID NO:1.

9. The method of item 8, wherein a plurality of target sites are operatively linked to the first nucleotide sequence.

10. The method of item 8, wherein a plurality of target sites are operatively linked to the second nucleotide sequence.

11. The method of item 8, wherein a plurality of target sites are operatively linked to both of the first and second nucleotide sequences.

12. The method of item 7, wherein the first nucleotide sequence comprises a cDNA sequence.

13. The method of item 7, wherein the second nucleotide sequence comprises a cDNA sequence.

14. The method of item 7, wherein both of the first and second nucleotide sequences comprise cDNA sequences.

15. The method of item 14, wherein the cDNA sequences encode antibody polypeptides.

16. The method of item 15 wherein the first cDNA sequence encodes an antibody heavy chain and the second cDNA sequence encodes an antibody light chain.

17. The method of item 7, wherein the protein further comprises a transcriptional activation domain.

18. The method of item 17, wherein the transcriptional activation domain is the VP16 domain.

## Claims

**1.** A method for inactivating endogenous cellular genes in a cell, the method comprising:

(a) inactivating a first gene in the cell; and
(b) expressing first and second fusion proteins in the cell, each of the first and second fusion proteins comprising a zinc finger DNA-binding domain and a nuclease, wherein the first and second fusion proteins bind to and cleave at a first site in the second gene;
thereby inactivating the first and second endogenous cellular genes .

**2.** The method of claim 1, wherein cleavage in the second gene is followed by non-homologous end joining.

**3.** The method of claim 1, further comprising expressing third and fourth fusion proteins in the cell, each of the third and fourth fusion proteins comprising a zinc finger protein and a nuclease, wherein the third and fourth fusion proteins bind to and cleave at a second site in the second gene.

**4.** The method of claim 1, further comprising contacting the cell with an exogenous nucleic acid sequence;
thereby resulting in integration of the exogenous nucleic acid sequence into the second gene.

**5.** The method of any of claims 1 to 4, wherein the first gene is *bax.*

**6.** The method of any of claims 1 to 4, wherein the first gene is *bac.*

**7.** The method of claim 5, wherein the first gene is *bax* and the second gene is bac.

**8.** The method of claim 6, wherein the first gene is bac and the second gene is *bax.*

**9.** The method of any of claims 1 to 4, wherein the first gene is glutamine synthetase.

**10.** The method of any of claims 1 to 4, wherein the first gene is dihydrofolate reductase.

**11.** The method of claim 9, wherein the first gene is glutamine synthetase and the second gene is dihydrofolate reductase.

**12.** The method of claim 10, wherein the first gene is dihydrofolate reductase and the second gene is glutamine synthetase.

**13.** An isolated cell produced by the method of any of claims 1-12.

**14.** The method of any of claims 1-12, wherein a third gene is inactivated, the method further comprising:

expressing fifth and sixth fusion proteins in the cell, each of the fifth and
sixth fusion proteins comprising a zinc finger DNA-binding domain and a nuclease, wherein the fifth and sixth fusion proteins bind to and cleave at a first site in the third gene;
thereby inactivating first, second and third genes.

**15.** The method of claim 14, wherein cleavage in the third gene is followed by non-homologous end joining.

**16.** The method of claim 14, further comprising expressing seventh and eighth fusion proteins in the cell, each of the seventh and eighth fusion proteins comprising a zinc finger protein and a nuclease, wherein the seventh and eighth fusion proteins bind to and cleave at a second site in the third gene.

**17.** The method of claim 14, further comprising contacting the cell with an exogenous nucleic acid sequence; thereby resulting in integration of the exogenous nucleic acid sequence into the third gene.

**18.** The method of any of claims 14-17, wherein the genes that are inactivated are the *bax, bac,* and α-1,6-fucosyltransferase genes.

**19.** An isolated cell produced by the method of any of claims 14 to 18.

# FIGURE 1

gctgtggaatgtgtgtcagttagggtgtggaaagtccccaggctccccagcaggcag
aagtatgcaaagcatgcatctcaattagtcagcaaccaggtgtggaaagtccccagg
ctccccagcaggcagaagtatgcaaagcatgcatctcaattagtcagcaaccatagt
cccgcccctaactccgcccatcccgcccctaactccgcccagttccgcccattctcc
gccccatggctgactaattttttttatttatgcagaggccgaggccgcctcggcctc
tgagctattccagaagtagtgaggaggcttttttggaggcctaggcttttgcaaaaa
gcttcgaggggctcgcatctctccttcacgcgcccgccgccctacctgaggccgcca
tccacgccggttgagtcgcgttctgccgcctcccgcctgtggtgcctcctgaactgc
gtccgccgtctaggtaagtttaaagctcaggtcgagaccgggcctttgtccggcgct
cccttggagcctacctagactcagccggctctccacgctttgcctgaccctgcttgc
tcaactctacgtctttgtttcgttttctgttctgcgccgttacagatccaagc

**SEQ ID NO:9**

# FIGURE 2

MA**PKKKRKV**GIDGVPGKKKQHICHIQGCGKVYG**QRSNLVR**HLRWHTGERP

50

FMCTWSYCGKRFT**RSDALSR**HKRTHTGEKKFACPECPKRFM**QSSDLRR**HI

100

KTHQNKKGGSGHRGRAPPTDVSLGDELHLDGEDVAMAHADALDDFDLDML

150

GDGDSPGPGFTPHDSAPYGALDMADFEFEQMFTDALGIDEYGGGRDYKDD

200

DDK

**SEQ ID NO:10**

# FIGURE 3

atggcc**cccaagaagaagaggaaggtg**ggaatcgatggggtaccgggcaa 50
gaagaagcagcacatctgccacatccagggctgtggtaaagtttacggcc 100
agcgctccaacctggtgcgccacctgcgctggcacaccggcgagaggcct 150
ttcatgtgtacatggtcctactgtggtaaacgcttcacccgctccgacgc 200
cctgtcccgccacaagcgtacccacaccggtgagaagaaatttgcttgtc 250
cggaatgtccgaagcgcttcatgcagtcctccgacctgcgccgccacatc 300
aagacccaccagaacaagaagggtggatccggccaccgcggccgcgcccc 350
cccgaccgatgtcagcctgggggacgagctccacttagacggcgaggacg 400
tggcgatggcgcatgccgacgcgctagacgatttcgatctggacatgttg 450
ggggacggggattccccgggtccgggatttacccccacgactccgcccc 500
ctacggcgctctggatatggccgacttcgagtttgagcagatgtttaccg 550
atgcccttggaattgacgagtacggtggcggccgcgactacaaggacgac 600
gatgacaag

**SEQ ID NO: 11**

# FIGURE 4

MA**PKKKRKV**GIDGVPGKKKQHICHIQGCGKVYG**QSSNLAR**HLRWHTGERP

50

FMCTWSYCGKRFT**RSDALTR**HKRTHTGEKKFACPECPKRFM**QSCDLTR**HI

100

KTHQNKKGGSGHRGRAPPTDVSLGDELHLDGEDVAMAHADALDDFDLDML

150

GDGDSPGPGFTPHDSAPYGALDMADFEFEQMFTDALGIDEYGGGRDYKDD

200

DDK

**SEQ ID NO:12**

# FIGURE 5

atggcc**cccaagaagaagaggaaggtg**ggaatcgatggggtaccgggcaa

50

gaagaagcagcacatctgccacatccagggctgtggtaaagtttacggcc

100

agtcctccaacctggcccgccacctgcgctggcacaccggcgagaggcct

150

ttcatgtgtacatggtcctactgtggtaaacgcttcacccgctccgacgc

200

cctgacccgccacaagcgtacccacaccggtgagaagaaatttgcttgtc

250

cggaatgtccgaagcgcttcatgcagtcctgcgacctgacccgccacatc

300

aagacccaccagaacaagaagggtggatccggccaccgcggccgcgcccc

350

cccgaccgatgtcagcctgggggacgagctccacttagacggcgaggacg

400

tggcgatggcgcatgccgacgcgctagacgatttcgatctggacatgttg 450

ggggacggggattccccgggtccgggatttaccccccacgactccgcccc 500

ctacggcgctctggatatggccgacttcgagtttgagcagatgtttaccg 550

atgcccttggaattgacgagtacggtggcggccgcgactacaaggacgac 600

gatgacaag

**SEQ ID NO:13**

# FIGURE 6

KKKQHICHIQGCGKVYGQRSNLVRHLRWHTGERPFMCTWSYCGKRFTRSDALSRHKR
THTGEKKFACPECPKRFMQSSDLTRHIKTHQNK (SEQ ID NO:14)

KKKQHICHIQGCGKVYGQSSNLARHLRWHTGERPFMCTWSYCGKRFTRSDALSRHKR
THTGEKKFACPECPKRFMQSSDLTRHIKTHQNK (SEQ ID NO:15)

KKKQHICHIQGCGKVYGQSSNLARHLRWHTGERPFMCTWSYCGKRFTRSDALSRHKR
THTGEKKFACPECPKRFMQSSDLRRHIKTHQNK (SEQ ID NO:16)

KKKQHICHIQGCGKVYGQSSNLARHLRWHTGERPFMCTWSYCGKRFTRSDALTRHKR
THTGEKKFACPECPKRFMQSCDLTRHIKTHQNK (SEQ ID NO:17)

KKKQHICHIQGCGKVYGDRSNLTRHLRWHTGERPFMCTWSYCGKRFTRSDALSRHKR
THTGEKKFACPECPKRFMQSSDLTRHIKTHQNK (SEQ ID NO:18)

KKKQHICHIQGCGKVYGQSSNLARHLRWHTGERPFMCTWSYCGKRFTRSDNLARHKR
THTGEKKFACPECPKRFMQSSDLRRHIKTHQNK (SEQ ID NO:19)

KKKQHICHIQGCGKVYGQSSNLARHLRWHTGERPFMCTWSYCGKRFTRSDNLARHKR
THTGEKKFACPECPKRFMQSSDLTRHIKTHQNK (SEQ ID NO:20)

KKKQHICHIQGCGKVYGDRSNLTRHLRWHTGERPFMCTWSYCGKRFTRSDALSRHKR
THTGEKKFACPECPKRFMQSSDLTRHIKTHQNK (SEQ ID NO:21)

KKKQHICHIQGCGKVYGQSSNLARHLRWHTGERPFMCTWSYCGKRFTRSDALTRHKR
THTGEKKFACPECPKRFMQSGDLTRHIKTHQNK (SEQ ID NO:22)

# FIGURE 7

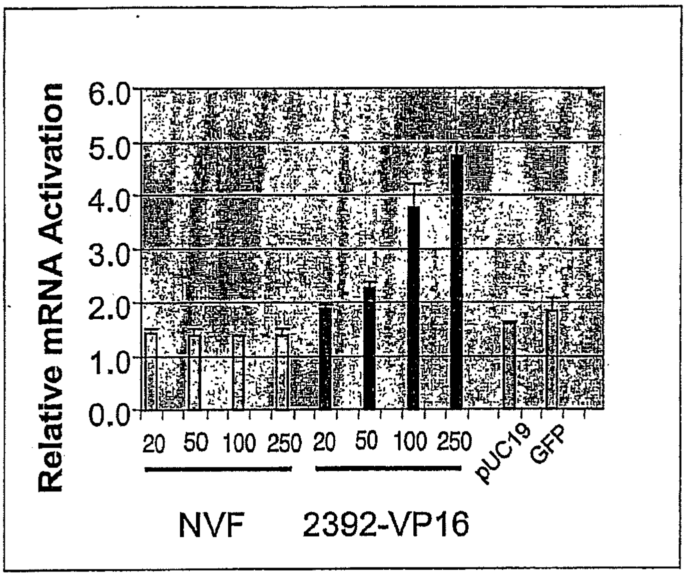
Relative mRNA Activation
6.0
5.0
4.0
3.0
2.0
1.0
0.0
20 50 100 250 20 50 100 250 pUC19 GFP
NVF 2392-VP16

# FIGURE 8

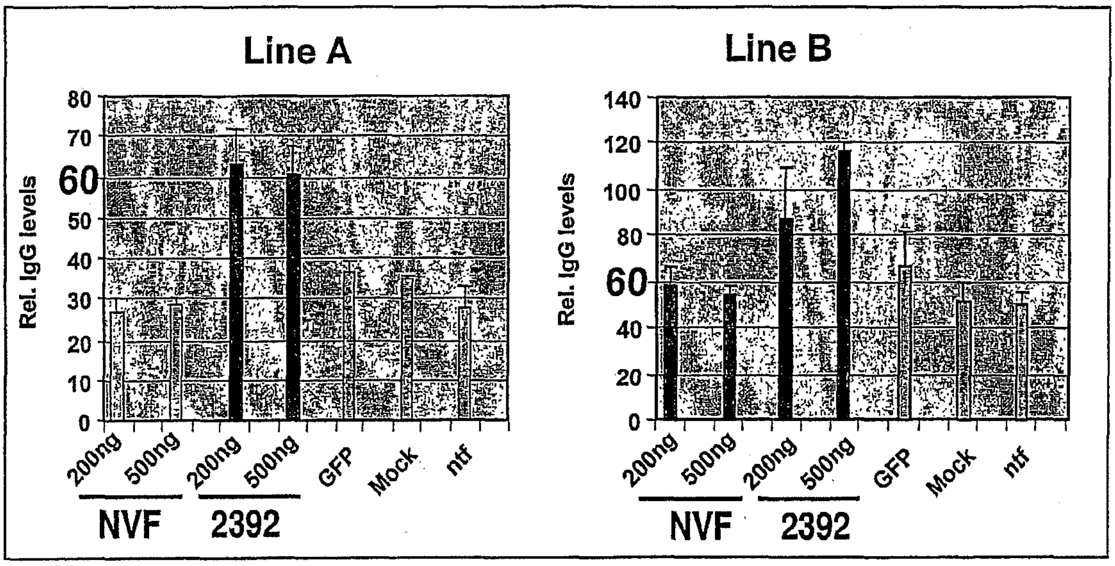
Line A
Rel. IgG levels
0
10
20
30
40
50
60
70
80
200ng
500ng
200ng
500ng
GFP
Mock
ntf
NVF
2392
Line B
Rel. IgG levels
0
20
40
60
80
100
120
140
200ng
500ng
200ng
500ng
GFP
Mock
ntf
NVF
2392

# FIGURE 9

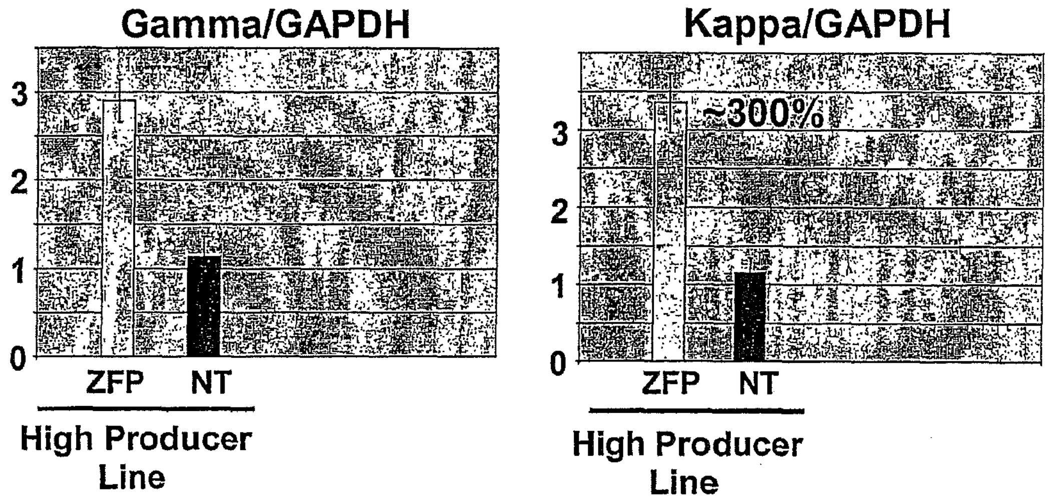
Gamma/GAPDH
3
2
1
0
ZFP
NT
High Producer Line
Kappa/GAPDH
~300%
3
2
1
0
ZFP
NT
High Producer Line

# FIGURE 10

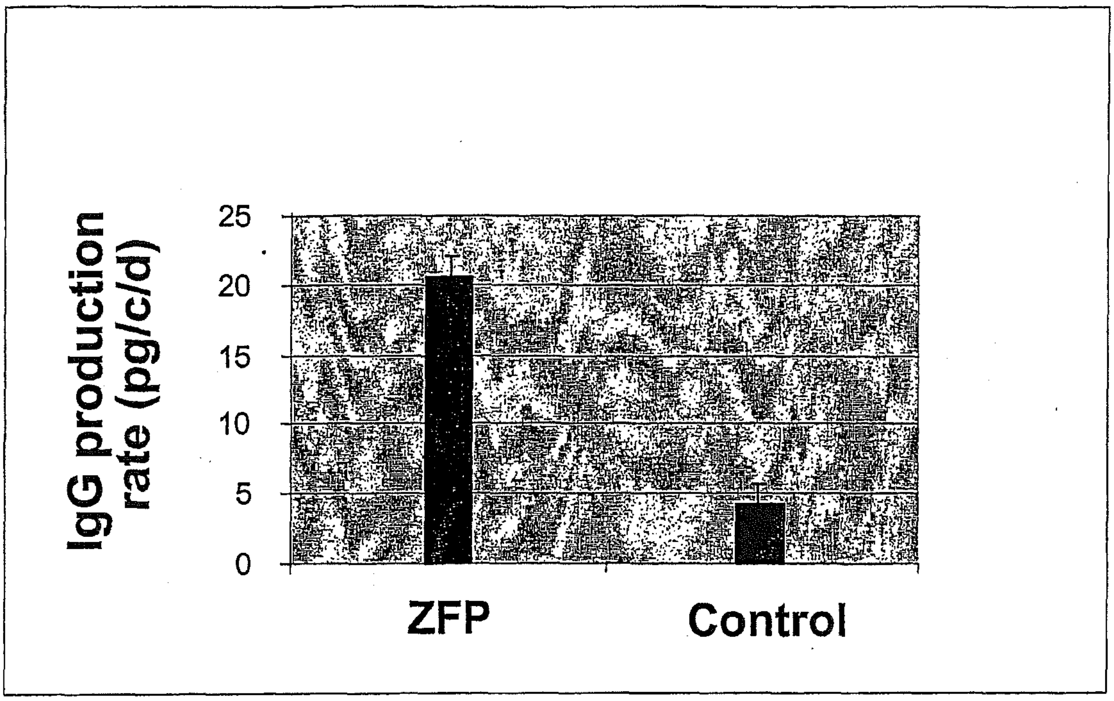

# FIGURE 11

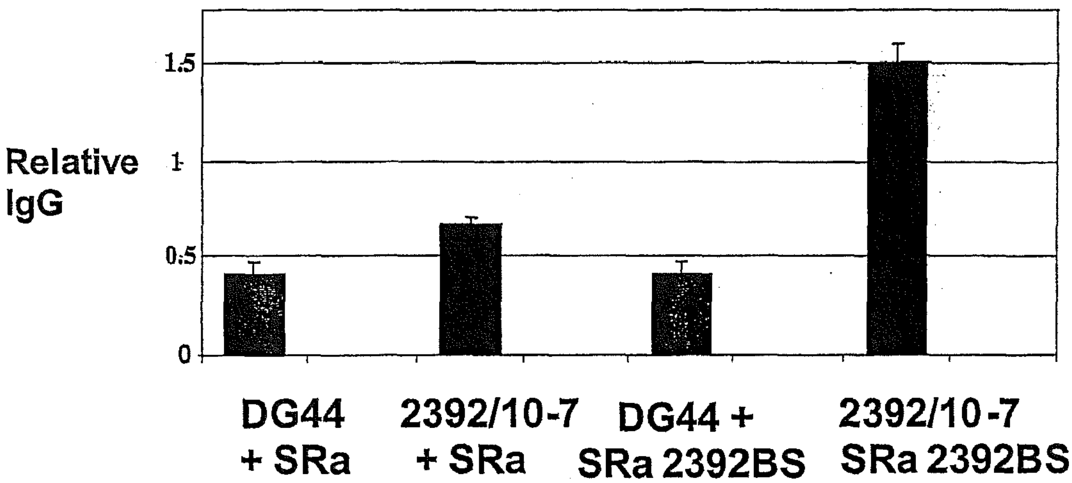
Relative IgG
1.5
1
0.5
0
DG44 + SRa
2392/10-7 + SRa
DG44 + SRa 2392BS
2392/10-7 SRa 2392BS

# FIGURE 12

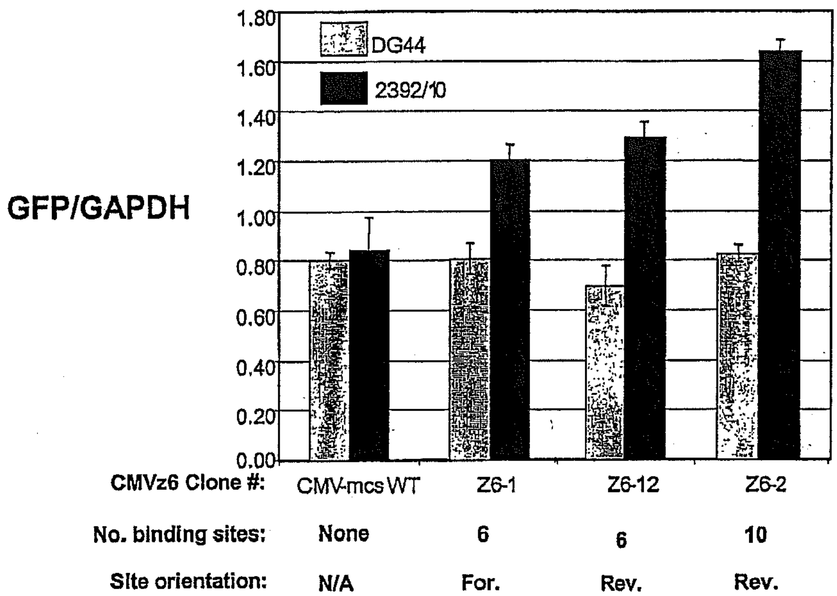
GFP/GAPDH
1.80
1.60
1.40
1.20
1.00
0.80
0.60
0.40
0.20
0.00
DG44
2392/10
CMVz6 Clone #:
CMV-mcs WT
Z6-1
Z6-12
Z6-2
No. binding sites:
None
6
6
10
Site orientation:
N/A
For.
Rev.
Rev.

# FIGURE 13

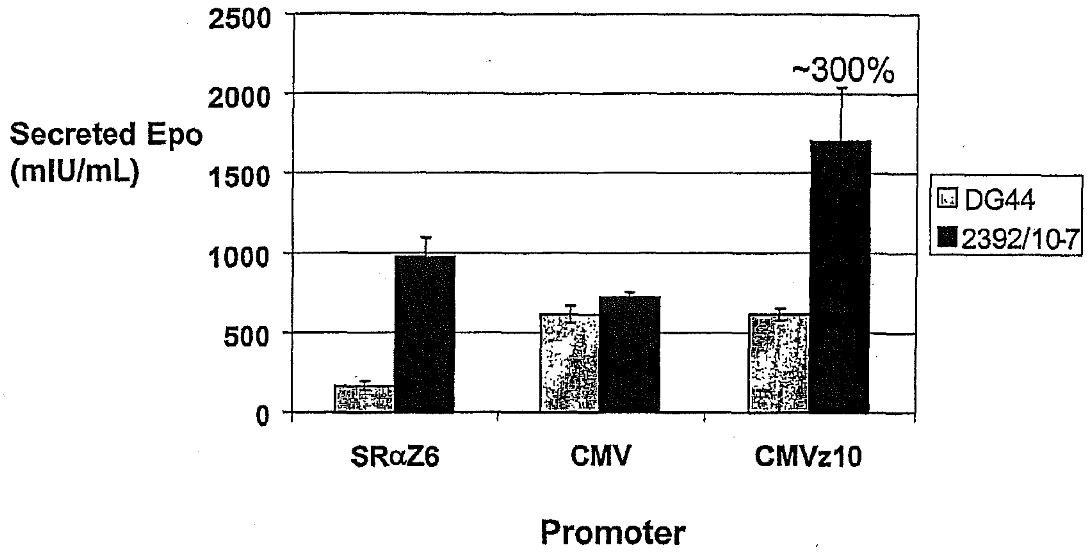

Secreted Epo (mIU/mL)
2500
2000
1500
1000
500
0
~300%
DG44
2392/10-7
SRαZ6
CMV
CMVz10
Promoter

Europäisches Patentamt
European Patent Office
Office européen des brevets

# EUROPEAN SEARCH REPORT

Application Number
EP 10 18 3733

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/160940 A1 (CASE CASEY C ET AL) 31 October 2002 (2002-10-31) * SEQ ID No: 7 has 90.00 % identity ( 90.00 % ungapped) over 90 (q:s=1-90:6-95) with subject GSP:ABG74227 disclosed in patent US2002160940 published on 20021031.; page 2 - page 3; claims 1-34; examples 1-13 * | 1-19 | INV. A61K48/00 C12N15/63 |
| X | US 6 607 882 B1 (COX, III GEORGE N ET AL) 19 August 2003 (2003-08-19) * claims 1-32 * | 1-19 | |
| X | US 2003/166141 A1 (CASE CASEY C ET AL) 4 September 2003 (2003-09-04) * claims 1-34 * | 1-19 | |
| X | JOUVENOT Y ET AL: "TARGETED REGULATION OF IMPRINTED GENES BY SYNTHETIC ZINC-FINGER TRANSCRIPTION FACTORS", GENE THERAPY, MACMILLAN PRESS LTD., BASINGSTOKE, GB, vol. 10, no. 6, March 2003 (2003-03), pages 513-522, XP009011657, ISSN: 0969-7128 * abstract; figure 1 * | 1-19 | TECHNICAL FIELDS SEARCHED (IPC): C12N A61K |

-/--

The present search report has been drawn up for all claims

| Place of search | Date of completion of the search | Examiner |
|---|---|---|
| Munich | 21 January 2011 | Vollbach, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 18 3733

| DOCUMENTS CONSIDERED TO BE RELEVANT | | | |
| --- | --- | --- | --- |
| Category | Citation of document with indication, where appropriate, of relevant passages | Relevant to claim | CLASSIFICATION OF THE APPLICATION (IPC) |
| X | LIU P-Q ET AL: "REGULATION OF AN ENDOGENOUS LOCUS USING A PANEL OF DESIGNED ZINC FINGER PRTEINS TARGETED TO ACESSIBLE CHROMATIN REGIONS ACTIVATION OF VASCULAR ENDOTHELIAL GROWTH FACTOR A", JOURNAL OF BIOLOGICAL CHEMISTRY, AMERICAN SOCIETY OF BIOLOCHEMICAL BIOLOGISTS, BIRMINGHAM,, US, vol. 276, no. 14, 6 April 2001 (2001-04-06), pages 11323-11334, XP000999288, ISSN: 0021-9258 * abstract; figures 2-7; table 2 * ----- | 1-19 | |
| | | | TECHNICAL FIELDS SEARCHED (IPC) |

The present search report has been drawn up for all claims

| Place of search | Date of completion of the search | Examiner |
| --- | --- | --- |
| Munich | 21 January 2011 | Vollbach, Silke |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 18 3733

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2011

| Patent document cited in search report | Publication date | Patent family member(s) | Publication date |
|---|---|---|---|
| US 2002160940 A1 | 31-10-2002 | AT 304792 T | 15-10-2005 |
| | | AU 745844 B2 | 11-04-2002 |
| | | AU 2847000 A | 01-08-2000 |
| | | CA 2323086 A1 | 20-07-2000 |
| | | DE 60022705 D1 | 27-10-2005 |
| | | DE 60022705 T2 | 14-06-2006 |
| | | DK 1061805 T3 | 09-01-2006 |
| | | EP 1061805 A1 | 27-12-2000 |
| | | ES 2250103 T3 | 16-04-2006 |
| | | GB 2348424 A | 04-10-2000 |
| | | JP 2002534104 T | 15-10-2002 |
| | | JP 2001231583 A | 28-08-2001 |
| | | WO 0041566 A1 | 20-07-2000 |
| | | US 6534261 B1 | 18-03-2003 |
| | | US 6607882 B1 | 19-08-2003 |
| | | US 6824978 B1 | 30-11-2004 |
| | | US 2003087817 A1 | 08-05-2003 |
| US 6607882 B1 | 19-08-2003 | AT 304792 T | 15-10-2005 |
| | | AU 745844 B2 | 11-04-2002 |
| | | AU 2847000 A | 01-08-2000 |
| | | CA 2323086 A1 | 20-07-2000 |
| | | DE 60022705 D1 | 27-10-2005 |
| | | DE 60022705 T2 | 14-06-2006 |
| | | DK 1061805 T3 | 09-01-2006 |
| | | EP 1061805 A1 | 27-12-2000 |
| | | ES 2250103 T3 | 16-04-2006 |
| | | GB 2348424 A | 04-10-2000 |
| | | JP 2002534104 T | 15-10-2002 |
| | | JP 2001231583 A | 28-08-2001 |
| | | WO 0041566 A1 | 20-07-2000 |
| | | US 6534261 B1 | 18-03-2003 |
| | | US 6824978 B1 | 30-11-2004 |
| | | US 2003087817 A1 | 08-05-2003 |
| | | US 2002160940 A1 | 31-10-2002 |
| US 2003166141 A1 | 04-09-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60610853 B
- US 60661841 B
- US 5168062 A
- US 5385839 A
- US 5272071 A
- US 5641670 A
- US 5733761 A
- US 5968502 A
- US 6361972 A
- US 6140081 A
- US 6453242 A
- US 6534261 A
- WO 9853058 A
- WO 9853059 A
- WO 9853060 A
- WO 02016536 A
- WO 03016496 A
- US 5789538 A
- US 5925523 A
- US 6007988 A
- US 6013453 A
- US 6200759 B
- WO 9519431 A
- WO 9606166 A
- WO 9853057 A
- WO 9854311 A
- WO 0027878 A
- WO 0160970 A
- WO 0188197 A
- WO 02099084 A
- US 6140466 A
- US 6242568 A
- US 6410248 A
- US 6479626 A
- US 6503717 A
- US 6706470 A
- US 6733970 A
- US 6746838 A
- US 20030044957 A
- US 20030068675 A
- US 20030104526 A
- US 20030108880 A
- US 20030166141 A
- US 20040091991 A
- WO 9853057 PCT
- WO 0153480 A
- WO 0277227 A
- US 5176996 A
- US 5422251 A
- US 5585245 A
- WO 9844350 A
- US 20020160940 A
- US 6453242 B
- US 6534261 B
- WO 9606166 PCT
- US 5436150 A
- US 5792640 A
- US 6265196 B
- US 20030232410 A
- WO 0387341 A
- WO 0104296 A
- WO 9927092 A
- US 6270990 B
- US 20020115215 A
- US 20030082552 A
- WO 0244376 A
- US 5834266 A
- US 5994313 A
- WO 9910508 A
- US 5364791 A
- US 5874534 A
- US 5935934 A
- US 91293204 A
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A
- US 9405700 W
- US 4797368 A
- WO 9324641 A
- US 5173414 A
- WO 9117424 PCT
- US 4957773 A
- US 4603044 A
- WO 0102553 A
- US 5614396 A
- WO 2005014791 A
- US 60702394 US
- US 20030044787 A

## Non-patent literature cited in the description


- **Takebe et al.** *Mol. Cell. Biol.,* 1988, vol. 8, 466-472
- **Sambrook et al.** MOLECULAR CLONING: A LABORATORY MANUAL. Cold Spring Harbor Laboratory Press, 1982
- MOLECULAR CLONING: A LABORATORY MANUAL. 2001
- **Ausubel et al.** CURRENT PROTOCOLS IN MOLECULAR BIOLOGY. John Wiley & Sons, 1987
- METHODS IN ENZYMOLOGY. Academic Press
- **Wolffe.** CHROMATIN STRUCTURE AND FUNCTION. Academic Press, 1998
- Chromatin. METHODS IN ENZYMOLOGY. Academic Press, vol. 304
- Chromatin Protocols. METHODS IN MOLECULAR BIOLOGY. Humana Press, 1999, vol. 119
- **Smith ; Waterman.** *Advances in Applied Mathematics,* 1981, vol. 2, 482-489
- **Dayhoff.** Atlas of Protein Sequences and Structure. ational Biomedical Research Foundation, vol. 3, 353-358
- **Gribskov.** *Nucl. Acids Res.,* 1986, vol. 14 (6), 6745-6763
- Wisconsin Sequence Analysis Package Program Manual. 1995
- Nucleic Acid Hybridization: A Practical Approach. IRL Press, 1985
- **Sambrook et al.** Molecular Cloning: A Laboratory Manual. Cold Spring Harbor, 1989
- **Fields et al.** *Nature,* 1989, vol. 340, 245-246
- **O'Shea.** *Science,* 1991, vol. 254, 539
- **Barahmand-Pour et al.** *Curr. Top. Microbiol. Immunol.,* 1996, vol. 211, 121-128
- **Klemm et al.** *Annu. Rev. Immunol.,* 1998, vol. 16, 569-592
- **Ho et al.** *Nature,* 1996, vol. 382, 822-826
- **Pomeranz et al.** *Biochem.,* 1998, vol. 37, 965
- **Goodrich et al.** *Cell,* 1996, vol. 84, 825-30
- **Barnes ; Adcock.** *Clin. Exp. Allergy,* 1995, vol. 25, 46-9
- **Roeder.** *Methods Enzymol.,* 1996, vol. 273, 165-71
- *Science,* 1995, vol. 269, 630
- **Rosen et al.** *J. Med. Chem.,* 1995, vol. 38, 4855-74
- **Wedel et al.** *Immunobiology,* 1995, vol. 193, 171-85
- **Meier.** *Eur. J. Endocrinol.,* 1996, vol. 134 (2), 158-9
- **Kaiser et al.** *Trends Biochem. Sci.,* 1996, vol. 21, 342-5
- **Utley et al.** *Nature,* vol. 394, 498-502
- **Simon.** *Nat. Genet.,* 1995, vol. 11, 9-11
- **Weiss et al.** *Exp. Hematol.,* vol. 23, 99-107
- **Goodrich ; Tjian.** *Curr. Opin. Cell Biol.,* 1994, vol. 6, 403-9
- **Hurley.** *Curr. Opin. Struct. Biol.,* 1996, vol. 6, 69-75
- **Barahmand-Pour et al.** *Curr. Top. Microbiol. Immunol.,* 1996, vol. 211, 121-8
- **Aso et al.** *J Clin. Invest.,* 1996, vol. 97, 1561-9
- **Thiesen et al.** *New Biologist,* 1990, vol. 2, 363-374
- **Margolin et al.** *PNAS,* 1994, vol. 91, 4509-4513
- **Pengue et al.** *Nucl. Acids Res.,* 1994, vol. 22, 2908-2914
- **Witzgall et al.** *PNAS,* 1994, vol. 91, 4514-4518
- **Friedman et al.** *Genes Dev.,* 1996, vol. 10, 2067-2078
- **Sommer et al.** *J. Biol. Chem.,* 1998, vol. 273, 6632-6642
- **Gupta et al.** *Oncogene,* 1998, vol. 16, 1149-1159
- **Queva et al.** *Oncogene,* 1998, vol. 16, 967-977
- **Larsson et al.** *Oncogene,* 1997, vol. 15, 737-748
- **Laherty et al.** *Cell,* 1997, vol. 89, 349-356
- **Cultraro et al.** *Mol Cell. Biol.,* vol. 17, 2353-2359
- **Ginsberg et al.** *Cancer Res.,* 1998, vol. 15, 3542-3546
- **Epstein et al.** *Mol. Cell. Biol.,* 1998, vol. 18, 4118-4130
- **Yan et al.** *PNAS,* 1998, vol. 95, 8298-8303
- **Liu et al.** *Cancer Gene Ther.,* 1998, vol. 5, 3-28
- **Sgouras et al.** *EMBO J.,* vol. 14, 4781-4793
- **Ayer et al.** *Mol. Cell. Biol.,* 1996, vol. 16, 5772-5781
- **Hagmann et al.** *J. Virol.,* 1997, vol. 71, 5952-5962
- **Torchia et al.** *Curr. Opin. Cell. Biol.,* 1998, vol. 10, 373-383
- **Bitko ; Barik.** *J. Virol.,* 1998, vol. 72, 5610-5618
- **Doyle ; Hunt.** *Neuroreport,* 1997, vol. 8, 2937-2942
- **Seipel et al.** *EMBO J.,* 1996, vol. 11, 4961-4968
- **Davis.** *Mol. Reprod. Dev.,* 1995, vol. 42, 459-67
- **Jackson et al.** *Adv. Second Messenger Phosphoprotein Res.,* 1993, vol. 28, 279-86
- **Boulikas.** *Crit. Rev. Eukaryot. Gene Expr.,* 1995, vol. 5, 1-77

- **Schonthal ; Semin.** *Cancer Biol.,* 1995, vol. 6, 239-48
- **Wang.** *Trends Biochem. Sci.,* 1994, vol. 19, 373-6
- The Jones and Bartlett Series in Biology. **Cooper.** Oncogenes. Jones and Bartlett Publishers, 1995
- **Waslylk et al.** *Eur. J. Biochem.,* 1993, vol. 211, 7-18
- **Crepieux et al.** *Crit. Rev. Oncog.,* 1994, vol. 5, 615-38
- **Ryan et al.** *Biochem. J.,* vol. 314, 713-21
- The Fos and Jun Families of Transcription Factors. 1994
- The max oncogene. **Hurlin et al.** Symp. Quant. Biol. Cold Spring Harb, vol. 59, 109-16
- **Kanei-Ishii et al.** *Curr. Top. Microbiol. Immunol.,* 1996, vol. 211, 89-98
- **Yew et al.** *Curr. Opin. Genet. Dev.,* 1993, vol. 3, 19-25
- **Vos.** *Curr. Opin. Cell Biol.,* 1992, vol. 4, 385-95
- **Sancar.** *Ann. Rev. Genet.,* 1995, vol. 29, 69-105
- **Lehmann.** *Genet. Eng.,* 1995, vol. 17, 1-19
- **Wood.** *Ann. Rev. Biochem.,* 1996, vol. 65, 135-67
- **Gangloff et al.** *Experientia,* 1994, vol. 50, 261-9
- **Sadowski.** *FASEB J.,* 1993, vol. 7, 760-7
- **Matson et al.** *Bioessays,* 1994, vol. 16, 13-22
- **Cheng.** *Curr. Opin. Struct, Biol.,* 1995, vol. 5, 4-10
- **Wolffe.** *Science,* 1996, vol. 272, 371-2
- **Van den Wyngaert et al.** *FEBS Lett.,* 1998, vol. 426, 283-289
- **Flynn et al.** *J. Mol. Biol.,* 1998, vol. 279, 101-116
- **Okano et al.** *Nucleic Acids Res.,* 1998, vol. 26, 2536-2540
- **Zardo ; Caiafa.** *J. Biol. Chem.,* 1998, vol. 273, 16517-16520
- **Jin ; Scotto.** *Mol. Cell. Biol.,* 1998, vol. 18, 4377-4384
- **Wolffe.** *Science,* 1996, vol. 272, 371-372
- **Taunton et al.** *Science,* 1996, vol. 272, 408-411
- **Hassig et al.** *PNAS,* 1998, vol. 95, 3519-3524
- **Jin ; Scotto.** *Mol. Cell. Biol,* vol. 18, 4377-4384
- **Syntichaki ; Thireos.** *J. Biol. Chem.,* 1998, vol. 273, 24414-24419
- **Sakaguchi et al.** *Genes Dev.,* 1998, vol. 12, 2831-2841
- **Martinez et al.** *J. Biol. Chem.,* 1998, vol. 273, 23781-23785
- **Hendrich et al.** *Mamm Genome,* 1999, vol. 10, 906-912
- **Damm et al.** *Nature,* 1989, vol. 339, 593-597
- **Evans.** *Int. J. Cancer Suppl.,* 1989, vol. 4, 26-28
- **Pain et al.** *New Biol.,* 1990, vol. 2, 284-294
- **Sap et al.** *Nature,* 1989, vol. 340, 242-244
- **Zenke et al.** *Cell,* 1988, vol. 52, 107-119
- **Zenke et al.** *Cell,* 1990, vol. 61, 1035-1049
- **Bird et al.** *Cell,* 1999, vol. 99, 451-454
- **Tyler et al.** *Cell,* 1999, vol. 99, 443-446
- **Knoepfler et al.** *Cell,* 1999, vol. 99, 447-450
- **Robertson et al.** *Nature Genet.,* 2000, vol. 25, 338-342
- **Chern et al.** *Plant Cell,* 1996, vol. 8, 305-321
- **Wu et al.** *Plant J.,* 2000, vol. 22, 19-27
- **Zhang et al.** *Annu. Rev. Physiol.,* 2000, vol. 62, 439-466
- **Sucov et al.** *Mol Neurobiol,* 1995, vol. 10 (2-3), 169-184
- **Guenther et al.** *Genes Dev,* 2000, vol. 14, 1048-1057
- **Urnov et al.** *EMBO J,* 2000, vol. 19, 4074-4090
- **Li et al.** *EMBO J,* 2000, vol. 19, 4342-4350
- **Underhill et al.** *J. Biol. Chem,* 2000, vol. 275, 40463-40470
- **Fondell et al.** *Genes Dev,* 1993, vol. 7 (7B), 1400-1410
- **Fondell et al.** *Cell,* 1996, vol. 16, 281-287
- **Collingwood et al.** *J. Mol. Endocrinol.,* 1999, vol. 23 (3), 255-275
- **Beug et al.** *Biochim Biophys Acta,* 1996, vol. 1288 (3), M35-47
- **Ciana et al.** *EMBO J.,* 1999, vol. 17, 7382-7394
- **Zazopoulos et al.** *Nature,* 1997, vol. 390, 311-315
- **Altincicek et al.** *J. Biol. Chem.,* 2000, vol. 275, 7662-7667
- **Lai et al.** *Oncogene,* 1999, vol. 18, 2091-2100
- **Lai et al.** *Mol. Cell. Biol.,* 1999, vol. 19, 6632-6641
- **Lai et al.** *Mol. Cell. Biol.,* 2001, vol. 21, 2918-2932
- **Cook et al.** *J. Biol. Chem.,* 1999, vol. 274, 29, 500-29504
- **Zhang et al.** *Mol. Cell. Biol.,* 2001, vol. 21, 5041-5049
- **LaPierre et al.** *J. Virol.,* 2001, vol. 75, 6062-6069
- **Sabbattini.** *EMBO J.,* 2001, vol. 20, 2812-2822
- **Maxon et al.** *Proc. Natl. Acad. Sci. USA,* 2001, vol. 98, 1495-1500
- **Fisher et al.** *Mol. Cell. Biol.,* 1996, vol. 16, 2670-2677
- **He et al.** *Proc. Natl. Acad. Sci. USA,* 1999, vol. 96, 10, 212-10217

- **Carreira et al.** *Mol. Cell. Biol.,* 1998, vol. 18, 5, 099-5108
- **Denisenko et al.** *Mol. Cell. Biol.,* 1998, vol. 18, 5634-5642
- **Garcia et al.** *EMBO J.,* 1999, vol. 18, 3404-3418
- **Satjin et al.** *Mol. Cell. Biol.,* 1997, vol. 17, 4105-4113
- **Gunster et al.** *Mol. Cell. Biol.,* 1997, vol. 17, 2326-2335
- **Hemenway et al.** *Oncogene,* 1998, vol. 16, 2541-2547
- **Trimarchi et al.** *Proc Natl. Acad. Sci. USA,* 2001, vol. 98, 1519-1524
- **Vlag et al.** *Nature Genet.,* 1999, vol. 23, 474-478
- **Richard et al.** *Mol. Cell. Biol.,* 1999, vol. 19, 777-787
- **Robyr et al.** *Mol. Endocrinol.,* 2000, vol. 14, 329-347
- **Collingwood et al.** *J. Mol. Endocrinol.,* 2000, vol. 23, 255-275
- **Leo.** *Gene,* 2000, vol. 245, 1-11
- **Manteuffel-Cymborowska.** *Acta Biochim. Pol.,* 1999, vol. 46, 77-89
- **McKenna et al.** *J. Steroid Biochem. Mol. Biol.,* vol. 69, 3-12
- **Malik et al.** *Trends Biochem.,* 2000, vol. 25, 277-283
- **Lemon et al.** *Curr. Opin. Genet. Dev.,* 1999, vol. 9, 499-504
- **Ogawa et al.** *Gene,* 2000, vol. 245, 21-29
- **Okanami et al.** *Genes Cells,* 1996, vol. 1, 87-99
- **Goff et al.** *Genes Dev.,* 1991, vol. 5, 298-309
- **Cho et al.** *Plant Mol. Biol.,* 1999, vol. 40, 419-429
- **Ulmason et al.** *Proc. Natl. Acad. Sci. USA,* 1999, vol. 96, 5844-5849
- **Sprenger-Haussels et al.** *Plant J.,* 2000, vol. 22, 1-8
- **Gong et al.** *Plant Mol. Biol.,* 1999, vol. 41, 33-44
- **Hobo et al.** *Proc. Natl. Acad. Sci. USA,* 1999, vol. 96, 15, 348-15353
- **Wang et al.** *Proc. Natl. Acad. Sci. USA,* 1994, vol. 91, 8180-8184
- **Wang et al.** *Gene Ther.,* 1997, vol. 4, 432-441
- **Tora et al.** *EMBO J.,* 1989, vol. 8, 1981-1986
- **Sambrook et al.** Molecular Cloning A Laboratory Manual. 1989
- **Kriegler.** Gene Transfer and Expression: A Laboratory Manual. 1990
- Current Protocols in Molecular Biology
- **Palva et al.** *Gene,* 1983, vol. 22, 229-235
- **Gossen ; Bujard.** *PNAS,* 1992, vol. 89, 5547
- **Oligino et al.** *Gene Ther.,* 1998, vol. 5, 491-496
- **Neering et al.** *Blood,* 1996, vol. 88, 1147-1155
- **Rendahl et al.** *Nat. Biotechnol.,* 1998, vol. 16, 757-761
- **Colley et al.** *J. Biol. Chem.,* 1989, vol. 264, 17619-17622
- Guide to Protein Purification. Methods in Enzymology. 1990, vol. 182
- **Morrison.** *J. Bact.,* 1977, vol. 132, 349-351
- **Clark-Curtiss ; Curtiss et al.** Methods in Enzymology. vol. 101, 347-362
- **Anderson.** *Science,* 1992, vol. 256, 808-813
- **Nabel ; Felgner.** *TIBTECH,* 1993, vol. 11, 211-217
- **Mitani ; Caskey.** *TIBTECH,* 1993, vol. 11, 162-166
- **Dillon.** *TIBTECH,* 1993, vol. 11, 167-175
- **Miller.** *Nature,* 1992, vol. 357, 455-460
- **Van Brunt.** *Biotechnology,* 1988, vol. 6 (10), 1149-1154
- **Vigne.** *Restorative Neurology and Neuroscience,* 1995, vol. 8, 35-36
- **Kremer ; Perricaudet.** *British Medical Bulletin,* 1995, vol. 51 (1), 31-44
- **Haddada et al.** Current Topics in Microbiology and Immunology. 1995
- **Yu et al.** *Gene Therapy,* 1994, vol. 1, 13-26
- **Crystal.** *Science,* 1995, vol. 270, 404-410
- **Blaese et al.** *Cancer Gene Ther.,* 1995, vol. 2, 291-297
- **Behr et al.** *Bioconjugate Chem.,* 1994, vol. 5, 382-389
- **Remy et al.** *Bioconjugate Chem.,* 1994, vol. 5, 647-654
- **Gao et al.** *Gene Therapy,* 1995, vol. 2, 710-722
- **Ahmad et al.** *Cancer Res.,* 1992, vol. 52, 4817-4820
- **Buchscher et al.** *J. Virol.,* 1992, vol. 66, 2731-2739
- **Johann et al.** *J. Virol.,* 1992, vol. 66, 1635-1640
- **Sommerfelt et al.** *Virol.,* 1990, vol. 176, 58-59
- **Wilson et al.** *J. Virol.,* 1989, vol. 63, 2374-2378
- **Miller et al.** *J. Virol.,* 1991, vol. 65, 2220-2224
- **West et al.** *Virology,* 1987, vol. 160, 38-47
- **Kotin.** *Human Gene Therapy,* 1994, vol. 5, 793-801
- **Muzyczka.** *J. Clin. Invest.,* 1994, vol. 94, 1351
- **Tratschin et al.** *Mol. Cell. Biol.,* 1985, vol. 5, 3251-3260
- **Tratschin et al.** *Mol. Cell. Biol.,* 1984, vol. 4, 2072-2081
- **Hermonat ; Muzyczka.** *PNAS,* 1984, vol. 81, 6466-6470

- **Samulski et al.** *J. Virol.,* 1989, vol. 63, 03822-3828
- **Dunbar et al.** *Blood,* 1995, vol. 85, 3048-305
- **Kohn et al.** *Nat. Med.,* 1995, vol. 1, 1017-102
- **Malech et al.** *PNAS,* 1997, vol. 94 (22), 12133-12138
- **Blaese et al.** *Science,* 1995, vol. 270, 475-480
- **Ellem et al.** *Immunol Immunother.,* 1997, vol. 44 (1), 10-20
- **Dranoff et al.** *Hum. Gene Ther.,* 1997, vol. 1, 111-2
- **Wagner et al.** *Lancet,* 1998, vol. 351, 91171702-3
- **Kearns et al.** *Gene Ther.,* 1996, vol. 9, 748-55
- **Sterman et al.** *Hum. Gene Ther.,* 1998, vol. 7, 1083-9
- **Rosenecker et al.** *Infection,* 1996, vol. 24, 1 5-10
- **Sterman et al.** *Hum. Gene Ther.,* 1998, vol. 9 (7), 1083-1089
- **Welsh et al.** *Hum. Gene Ther.,* 1995, vol. 2, 205-18
- **Alvarez et al.** *Hum. Gene Ther.,* 1997, vol. 5, 597-613
- **Topf et al.** *Gene Ther.,* 1998, vol. 5, 507-513
- **Sterman et al.** *Hum. Gene Ther.,* 1998, vol. 7, 1083-1089
- **Han et al.** *Proc. Natl. Acad. Sci. USA,* 1995, vol. 92, 9747-9751
- **Freshney et al.** Culture of Animal Cells, A Manual of Basic Technique
- **Inaba et al.** *J. Exp. Med.,* 1992, vol. 176, 1693-1702
- Remington's Pharmaceutical Sciences. 1989
- **Weissbach ; Weissbach.** Methods for Plant Molecular Biology. Academic Press, 1988, 421-463
- **Grierson ; Corey.** Plant Molecular Biology. Blackie, 1988
- **Klein et al.** *Nature,* 1987, vol. 327, 70-73
- **Horsch et al.** *Science,* 1984, vol. 233, 496-498
- **Fraley et al.** *Proc. Nat'l. Acad. Sci. USA,* 1983, vol. 80, 4803
- **Bevan.** *Nuc. Acid Res.,* 1984, vol. 12, 8711-8721
- **Horsch et al.** *Science,* 1985, vol. 227, 1229-1231
- **Bevan et al.** *Ann. Rev. Genet,* 1982, vol. 16, 357-384
- **Rogers et al.** *Methods Enzymol.,* 1986, vol. 118, 627-641
- **Hernalsteen et al.** *EMBO J,* 1984, vol. 3, 3039-3041
- **Hooykass-Van Slogteren et al.** *Nature,* 1984, vol. 311, 763-764
- **Grimsley et al.** *Nature,* 1987, vol. 325, 1677-179
- **Boulton et al.** *Plant Mol. Biol.,* vol. 12, 31-40
- **Gould et al.** *Plant Physiol.,* 1991, vol. 95, 426-434
- **Paszkowski et al.** *EMBO J,* 1984, vol. 3, 2717-2722
- **Potrykus et al.** *Molec. Gen. Genet.,* 1985, vol. 199, 169-177
- **Fromm et al.** *Proc. Nat. Acad. Sci. USA,* 1985, vol. 82, 5824-5828
- **Shimamoto.** *Nature,* 1989, vol. 338, 274-276
- **D'Halluin et al.** *Plant Cell,* 1992, vol. 4, 1495-1505
- **Kaeppler et al.** *Plant Cell Reporter,* 1990, vol. 9, 415-418
- **Klein et al.** *Proc. Nat. Acad. Sci. USA,* 1988, vol. 85, 4305-4309
- **Gordon-Kamm et al.** *Plant Cell,* vol. 2, 603-618
- Protoplasts Isolation and Culture. **Evans et al.** Handbook of Plant Cell Culture. Macmillian Publishing Company, 1983, 124-176
- **Binding.** Regeneration of Plants, Plant Protoplasts. CRC Press, 1985, 21-73
- **Klee et al.** *Ann. Rev. of Plant Phys.,* 1987, vol. 38, 467-486
- **Prochiantz.** *Current Opinion in Neurobiology,* 1996, vol. 6, 629-634
- **Lin et al.** *J. Biol. Chem.,* 1995, vol. 270 (1), 4253-14258
- **Fahraeus et al.** *Current Biology,* 1996, vol. 6, 84
- **Derossi et al.** *J. Biol. Chem.,* 1994, vol. 269, 10444
- **Elliot ; O'Hare.** *Cell,* 1997, vol. 88, 223-233
- **Yeh et al.** *Molecular Therapy,* 2003, vol. 7 (5), S461
- **Arora et al.** *J. Biol. Chem.,* 1993, vol. 268, 3334-3341
- **Perelle et al.** *Infect. Immun.,* 1993, vol. 61, 5147-5156
- **Stenmark et al.** *J. Cell Biol.,* 1991, vol. 113, 1025-1032
- **Donnelly et al.** *PNAS,* 1993, vol. 90, 3530-3534
- **Carbonetti et al.** *Abstr. Annu. Meet. Am. Soc. Microbiol.,* 1995, vol. 95, 295
- **Sebo et al.** *Infect. Immun.,* 1995, vol. 63, 3851-3857
- **Klimpel et al.** *PNAS U.S.A.,* 1992, vol. 89, 10277-10281
- **Novak et al.** *J. Biol. Chem.,* 1992, vol. 267, 17186-17193
- *PNAS,* 1987, vol. 84, 7851
- *Biochemistry,* 1989, vol. 28, 908
- **Szoka et al.** *Ann. Rev. Biophys. Bioeng.,* 1980, vol. 9, 467
- **Deamer ; Bangham.** *Biochim. Biophys. Acta,* 1976, vol. 443, 629-634
- **Fraley et al.** *PNAS,* 1979, vol. 76, 3348-3352

- **Hope et al.** *Biochim. Biophys. Acta,* 1985, vol. 812, 55-6S
- **Mayer et al.** *Biochim. Biophys. Acta,* 1986, vol. 858, 161-168
- **Williams et al.** *PNAS,* 1988, vol. 85, 242-246
- Liposomes. 1983
- **Hope et al.** *Chem. Phys. Lip.,* 1986, vol. 40, 89
- **Gregoriadis.** *Liposome Technology,* 1984
- **Lasic.** *Liposomes: from Physics to Applications,* 1993
- **Renneisen et al.** *J. Biol. Chem.,* 1990, vol. 265, 16337-16342
- **Leonetti et al.** *PNAS,* 1990, vol. 87, 2448-2451
- Remington's Pharmaceutical Sciences. 1985
- **Liao et al.** *Science,* 1986, vol. 233, 364-367
- **Lu et al.** *Biochemistry,* 2000, vol. 39, 13575-13583
- **Premzl et al.** *Gene,* 2003, vol. 314, 89-102
- **Joung et al.** *Proc. Natl. Acad. Sci. USA,* 2000, vol. 97, 7382-7287

SEQUENCE LISTING

<110> LI, Xiao-Yong
JAMIESON, Andrew
BARTSEVICH, Victor
COLLINGWOOD, Trevor

<120> COMPOSITIONS AND METHODS FOR PROTEIN PRODUCTION

<130> 8325-0043.44 (S43-EP)

<140> 05794863.0
<141> 2005-09-08

<150> PCT/US2005/032157
<151> 2005-09-08

<150> 60/610,853
<151> 2004-09-16

<150> 60/661,841
<151> 2005-03-15

<160> 25

<170> PatentIn version 3.3

<210> 1
<211> 9
<212> DNA
<213> Artificial Sequence

<220>
<223> chemically synthesized target sequence

<400> 1
gctgtggaa
9

<210> 2
<211> 90
<212> PRT
<213> Artificial Sequence

<220>
<223> chemically synthesized three-finger zinc finger protein SBS2392/00

<400> 2

Lys Lys Lys Gln His Ile Cys His Ile Gln Gly Cys Gly Lys Val Tyr
1 5 10 15

Gly Gln Arg Ser Asn Leu Val Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Ser Arg His Lys Arg Thr His Thr Gly Glu Lys Lys
50 55 60

Phe Ala Cys Pro Glu Cys Pro Lys Arg Phe Met Gln Ser Ser Asp Leu
65 70 75 80

Arg Arg His Ile Lys Thr His Gln Asn Lys
85 90

<210> 3
<211> 89
<212> PRT
<213> Artificial Sequence

<220>
<223> chemically synthesized zinc finger motif

<220>
<221> MISC_FEATURE
<222> (2)..(5)
<223> Xaa = any amino acid

<220>
<221> MISC_FEATURE
<222> (4)..(5)
<223> Xaa may be present or absent

<220>
<221> MISC_FEATURE
<222> (7)..(11)
<223> Xaa = any amino acid

<220>
<221> MISC_FEATURE
<222> (20)..(24)
<223> Xaa = any amino acid

<220>
<221> MISC_FEATURE

<222> (23)..(24)
<223> Xaa may be present or absent

<220>
<221> MISC_FEATURE
<222> (26)..(32)
<223> Xaa = any amino acid

<220>
<221> MISC_FEATURE
<222> (34)..(37)
<223> Xaa = any amino acid

<220>
<221> MISC_FEATURE
<222> (36)..(37)
<223> Xaa may be present or absent

<220>
<221> MISC_FEATURE
<222> (39)..(43)
<223> Xaa = any amino acid

<220>
<221> MISC_FEATURE
<222> (52)..(56)
<223> Xaa = any amino acid

<220>
<221> MISC_FEATURE
<222> (55)..(56)
<223> Xaa may be present or absent

<220>
<221> MISC_FEATURE
<222> (58)..(64)
<223> Xaa = any amino acid

<220>
<221> MISC_FEATURE
<222> (66)..(69)
<223> Xaa = any amino acid

<220>
<221> MISC_FEATURE
<222> (68)..(69)
<223> Xaa may be present or absent

<220>
<221> MISC_FEATURE
<222> (71)..(75)
<223> Xaa = any amino acid

<220>
<221> MISC_FEATURE
<222> (84)..(88)
<223> Xaa = any amino acid

<220>
<221> MISC_FEATURE
<222> (87)..(88)
<223> Xaa may be present or absent

<400> 3

Cys Xaa Xaa Xaa Xaa Cys Xaa Xaa Xaa Xaa Xaa Gln Arg Ser Asn Leu
1               5                   10                  15

Val Arg His Xaa Xaa Xaa Xaa Xaa His Xaa Xaa Xaa Xaa Xaa Xaa Xaa
            20                  25                  30

Cys Xaa Xaa Xaa Xaa Cys Xaa Xaa Xaa Xaa Xaa Arg Ser Asp Ala Leu
        35                  40                  45

Ser Arg His Xaa Xaa Xaa Xaa Xaa His Xaa Xaa Xaa Xaa Xaa Xaa Xaa
    50                  55                  60

Cys Xaa Xaa Xaa Xaa Cys Xaa Xaa Xaa Xaa Xaa Gln Ser Ser Asp Leu
65                  70                  75                  80

Arg Arg His Xaa Xaa Xaa Xaa Xaa His
                85

<210> 4
<211> 7
<212> PRT
<213> Artificial Sequence

<220>
<223> chemically synthesized zinc finger

<220>
<221> misc_feature
<222> (2)..(3)
<223> Xaa can be any naturally occurring amino acid

<220>
<221> misc_feature
<222> (5)..(6)

<223> Xaa can be any naturally occurring amino acid

<400> 4

Gln Xaa Xaa Asn Xaa Xaa Arg
1 5

<210> 5
<211> 7
<212> PRT
<213> Artificial Sequence

<220>
<223> chemically synthesized zinc finger

<220>
<221> misc_feature
<222> (2)..(3)
<223> Xaa can be any naturally occurring amino acid

<220>
<221> misc_feature
<222> (5)..(6)
<223> Xaa can be any naturally occurring amino acid

<400> 5

Arg Xaa Xaa Ala Xaa Xaa Arg
1 5

<210> 6
<211> 7
<212> PRT
<213> Artificial Sequence

<220>
<223> chemically synthesized zinc finger

<220>
<221> misc_feature
<222> (2)..(3)
<223> Xaa can be any naturally occurring amino acid

<220>
<221> misc_feature
<222> (5)..(6)
<223> Xaa can be any naturally occurring amino acid

<400> 6

Gln Xaa Xaa Asp Xaa Xaa Arg
1 5

<210> 7
<211> 90
<212> PRT
<213> Artificial Sequence

<220>
<223> chemically synthesized zinc finger protein SBS2392/10

<400> 7

Lys Lys Lys Gln His Ile Cys His Ile Gln Gly Cys Gly Lys Val Tyr
1 5 10 15

Gly Gln Ser Ser Asn Leu Ala Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Thr Arg His Lys Arg Thr His Thr Gly Glu Lys Lys
50 55 60

Phe Ala Cys Pro Glu Cys Pro Lys Arg Phe Met Gln Ser Cys Asp Leu
65 70 75 80

Thr Arg His Ile Lys Thr His Gln Asn Lys
85 90

<210> 8
<211> 89
<212> PRT
<213> Artificial Sequence

<220>
<223> chemically synthesized zinc finger motif

<220>
<221> MISC_FEATURE
<222> (2)..(5)
<223> Xaa = any amino acid

<220>
<221> MISC_FEATURE
<222> (4)..(5)
<223> Xaa may be present or absent

<220>
<221> MISC_FEATURE
<222> (7)..(11)
<223> Xaa = any amino acid

<220>
<221> MISC_FEATURE
<222> (20)..(24)
<223> Xaa = any amino acid

<220>
<221> MISC_FEATURE
<222> (23)..(24)
<223> Xaa may be present or absent

<220>
<221> MISC_FEATURE
<222> (26)..(32)
<223> Xaa = any amino acid

<220>
<221> MISC_FEATURE
<222> (34)..(37)
<223> Xaa = any amino acid

<220>
<221> MISC_FEATURE
<222> (36)..(37)
<223> Xaa may be present or absent

<220>
<221> MISC_FEATURE
<222> (39)..(43)
<223> Xaa = any amino acid

<220>
<221> MISC_FEATURE
<222> (52)..(56)
<223> Xaa = any amino acid

<220>
<221> MISC_FEATURE
<222> (55)..(56)
<223> Xaa may be present or absent

<220>
<221> MISC_FEATURE
<222> (58)..(64)

<223> Xaa = any amino acid

<220>
<221> MISC_FEATURE
<222> (65)..(69)
<223> Xaa = any amino acid

<220>
<221> MISC_FEATURE
<222> (68)..(69)
<223> Xaa may be present or absent

<220>
<221> MISC_FEATURE
<222> (71)..(75)
<223> Xaa = any amino acid

<220>
<221> MISC_FEATURE
<222> (84)..(88)
<223> Xaa = any amino acid

<220>
<221> MISC_FEATURE
<222> (87)..(88)
<223> Xaa may be present or absent

<400> 8

```
Cys Xaa Xaa Xaa Xaa Cys Xaa Xaa Xaa Xaa Xaa Gln Ser Ser Asn Leu
1               5                   10                  15

Ala Arg His Xaa Xaa Xaa Xaa Xaa His Xaa Xaa Xaa Xaa Xaa Xaa Xaa
            20                  25                  30

Cys Xaa Xaa Xaa Xaa Cys Xaa Xaa Xaa Xaa Xaa Arg Ser Asp Ala Leu
        35                  40                  45

Thr Arg His Xaa Xaa Xaa Xaa Xaa His Xaa Xaa Xaa Xaa Xaa Xaa Xaa
    50                  55                  60

Cys Xaa Xaa Xaa Xaa Cys Xaa Xaa Xaa Xaa Xaa Gln Ser Cys Asp Leu
65                  70                  75                  80

Thr Arg His Xaa Xaa Xaa Xaa Xaa His
                85
```

<210> 9
<211> 623
<212> DNA
<213> Artificial Sequence

<220>
<223> chemically synthesized SR-alpha promoter

<400> 9
gctgtggaat gtgtgtcagt tagggtgtgg aaagtcccca ggctccccag caggcagaag 60

tatgcaaagc atgcatctca attagtcagc aaccaggtgt ggaaagtccc caggctcccc 120

agcaggcaga agtatgcaaa gcatgcatct caattagtca gcaaccatag tcccgcccct 180

aactccgccc atcccgcccc taactccgcc cagttccgcc cattctccgc cccatggctg 240

actaattttt tttatttatg cagaggccga ggccgcctcg gcctctgagc tattccagaa 300

gtagtgagga ggcttttttg gaggcctagg cttttgcaaa aagcttcgag ggctcgcat 360

ctctccttca cgcgcccgcc gccctacctg aggccgccat ccacgccggt tgagtcgcgt 420

tctgccgcct cccgcctgtg gtgcctcctg aactgcgtcc gccgtctagg taagtttaaa 480

gctcaggtcg agaccgggcc tttgtccggc gctcccttgg agcctaccta gactcagccg 540

gctctccacg ctttgcctga ccctgcttgc tcaactctac gtctttgttt cgttttctgt 600

tctgcgccgt tacagatcca agc 623

<210> 10
<211> 203
<212> PRT
<213> Artificial Sequence

<220>
<223> chemically synthesized sequence of the 2392/00 protein

<400> 10

```
Met Ala Pro Lys Lys Lys Arg Lys Val Gly Ile Asp Gly Val Pro Gly
1               5                   10                  15

Lys Lys Lys Gln His Ile Cys His Ile Gln Gly Cys Gly Lys Val Tyr
            20                  25                  30

Gly Gln Arg Ser Asn Leu Val Arg His Leu Arg Trp His Thr Gly Glu
        35                  40                  45

Arg Pro Phe Met Cys Thr Trp Ser Tyr Cys Gly Lys Arg Phe Thr Arg
    50                  55                  60

Ser Asp Ala Leu Ser Arg His Lys Arg Thr His Thr Gly Glu Lys Lys
65                  70                  75                  80

Phe Ala Cys Pro Glu Cys Pro Lys Arg Phe Met Gln Ser Ser Asp Leu
                85                  90                  95

Arg Arg His Ile Lys Thr His Gln Asn Lys Lys Gly Gly Ser Gly His
            100                 105                 110

Arg Gly Arg Ala Pro Pro Thr Asp Val Ser Leu Gly Asp Glu Leu His
        115                 120                 125

Leu Asp Gly Glu Asp Val Ala Met Ala His Ala Asp Ala Leu Asp Asp
    130                 135                 140

Phe Asp Leu Asp Met Leu Gly Asp Gly Asp Ser Pro Gly Pro Gly Phe
145                 150                 155                 160

Thr Pro His Asp Ser Ala Pro Tyr Gly Ala Leu Asp Met Ala Asp Phe
                165                 170                 175

Glu Phe Glu Gln Met Phe Thr Asp Ala Leu Gly Ile Asp Glu Tyr Gly
            180                 185                 190

Gly Gly Arg Asp Tyr Lys Asp Asp Asp Asp Lys
        195                 200
```

<210>  11

<211> 609
<212> DNA
<213> Artificial Sequence

<220>
<223> chemically synthesized sequence of a polynucleotide encoding the
2392/00 protein

<400> 11
atggccccca agaagaagag gaaggtggga atcgatgggg taccgggcaa gaagaagcag 60

cacatctgcc acatccaggg ctgtggtaaa gtttacggcc agcgctccaa cctggtgcgc 120

cacctgcgct ggcacaccgg cgagaggcct ttcatgtgta catggtccta ctgtggtaaa 180

cgcttcaccc gctccgacgc cctgtcccgc cacaagcgta cccacaccgg tgagaagaaa 240

tttgcttgtc cggaatgtcc gaagcgcttc atgcagtcct ccgacctgcg ccgccacatc 300

aagacccacc agaacaagaa gggtggatcc ggccaccgcg gccgcgcccc cccgaccgat 360

gtcagcctgg gggacgagct ccacttagac ggcgaggacg tggcgatggc gcatgccgac 420

gcgctagacg atttcgatct ggacatgttg ggggacgggg attccccggg tccgggattt 480

accccccacg actccgcccc ctacggcgct ctggatatgg ccgacttcga gtttgagcag 540

atgtttaccg atgcccttgg aattgacgag tacggtggcg gccgcgacta caaggacgac 600

gatgacaag 609

<210> 12
<211> 203
<212> PRT
<213> Artificial Sequence

<220>
<223> chemically synthesized sequence of the 2392/10 protein

<400> 12

Met Ala Pro Lys Lys Lys Arg Lys Val Gly Ile Asp Gly Val Pro Gly
1 5 10 15

Lys Lys Lys Gln His Ile Cys His Ile Gln Gly Cys Gly Lys Val Tyr
20 25 30

Gly Gln Ser Ser Asn Leu Ala Arg His Leu Arg Trp His Thr Gly Glu
35 40 45

Arg Pro Phe Met Cys Thr Trp Ser Tyr Cys Gly Lys Arg Phe Thr Arg
50 55 60

Ser Asp Ala Leu Thr Arg His Lys Arg Thr His Thr Gly Glu Lys Lys
65 70 75 80

Phe Ala Cys Pro Glu Cys Pro Lys Arg Phe Met Gln Ser Cys Asp Leu
85 90 95

Thr Arg His Ile Lys Thr His Gln Asn Lys Lys Gly Gly Ser Gly His
100 105 110

Arg Gly Arg Ala Pro Pro Thr Asp Val Ser Leu Gly Asp Glu Leu His
115 120 125

Leu Asp Gly Glu Asp Val Ala Met Ala His Ala Asp Ala Leu Asp Asp
130 135 140

Phe Asp Leu Asp Met Leu Gly Asp Gly Asp Ser Pro Gly Pro Gly Phe
145 150 155 160

Thr Pro His Asp Ser Ala Pro Tyr Gly Ala Leu Asp Met Ala Asp Phe
165 170 175

Glu Phe Glu Gln Met Phe Thr Asp Ala Leu Gly Ile Asp Glu Tyr Gly
180 185 190

Gly Gly Arg Asp Tyr Lys Asp Asp Asp Asp Lys
195 200

<210> 13
<211> 609
<212> DNA
<213> Artificial Sequence

<220>
<223> chemically synthesized sequence of a polynucleotide encoding the 2392/10 protein

<400> 13
atggccccca agaagaagag gaaggtggga atcgatgggg taccgggcaa gaagaagcag 60

cacatctgcc acatccaggg ctgtggtaaa gtttacggcc agtcctccaa cctggcccgc 120

cacctgcgct ggcacaccgg cgagaggcct ttcatgtgta catggtccta ctgtggtaaa 180

cgcttcaccc gctccgacgc cctgacccgc cacaagcgta cccacaccgg tgagaagaaa 240

tttgcttgtc cggaatgtcc gaagcgcttc atgcagtcct gcgacctgac ccgccacatc 300

aagacccacc agaacaagaa gggtggatcc ggccaccgcg gccgcgcccc cccgaccgat 360

gtcagcctgg gggacgagct ccacttagac ggcgaggacg tggcgatggc gcatgccgac 420

gcgctagacg atttcgatct ggacatgttg ggggacgggg attccccggg tccgggattt 480

acccccacg actccgcccc ctacggcgct ctggatatgg ccgacttcga gtttgagcag 540

atgtttaccg atgcccttgg aattgacgag tacggtggcg gccgcgacta caaggacgac 600

gatgacaag 609

<210> 14
<211> 90
<212> PRT
<213> Artificial Sequence

<220>
<223> chemically synthesized zinc finger

<400> 14

Lys Lys Lys Gln His Ile Cys His Ile Gln Gly Cys Gly Lys Val Tyr
1               5                   10                  15

Gly Gln Arg Ser Asn Leu Val Arg His Leu Arg Trp His Thr Gly Glu
            20                  25                  30

Arg Pro Phe Met Cys Thr Trp Ser Tyr Cys Gly Lys Arg Phe Thr Arg
        35                  40                  45

Ser Asp Ala Leu Ser Arg His Lys Arg Thr His Thr Gly Glu Lys Lys
    50                  55                  60

Phe Ala Cys Pro Glu Cys Pro Lys Arg Phe Met Gln Ser Ser Asp Leu
65                  70                  75                  80

Thr Arg His Ile Lys Thr His Gln Asn Lys
                85                  90

<210> 15
<211> 90
<212> PRT
<213> Artificial Sequence

<220>
<223> chemically synthesized zinc finger

<400> 15

Lys Lys Lys Gln His Ile Cys His Ile Gln Gly Cys Gly Lys Val Tyr
1               5                   10                  15

Gly Gln Ser Ser Asn Leu Ala Arg His Leu Arg Trp His Thr Gly Glu
            20                  25                  30

Arg Pro Phe Met Cys Thr Trp Ser Tyr Cys Gly Lys Arg Phe Thr Arg
        35                  40                  45

Ser Asp Ala Leu Ser Arg His Lys Arg Thr His Thr Gly Glu Lys Lys
    50                  55                  60

Phe Ala Cys Pro Glu Cys Pro Lys Arg Phe Met Gln Ser Ser Asp Leu

65 70 75 80

Thr Arg His Ile Lys Thr His Gln Asn Lys
85 90

<210> 16
<211> 90
<212> PRT
<213> Artificial Sequence

<220>
<223> chemically synthesized zinc finger

<400> 16

Lys Lys Lys Gln His Ile Cys His Ile Gln Gly Cys Gly Lys Val Tyr
1 5 10 15

Gly Gln Ser Ser Asn Leu Ala Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Ser Arg His Lys Arg Thr His Thr Gly Glu Lys Lys
50 55 60

Phe Ala Cys Pro Glu Cys Pro Lys Arg Phe Met Gln Ser Ser Asp Leu
65 70 75 80

Arg Arg His Ile Lys Thr His Gln Asn Lys
85 90

<210> 17
<211> 90
<212> PRT
<213> Artificial Sequence

<220>
<223> chemically synthesized zinc finger

<400> 17

Lys Lys Lys Gln His Ile Cys His Ile Gln Gly Cys Gly Lys Val Tyr
1 5 10 15

Gly Gln Ser Ser Asn Leu Ala Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Thr Arg His Lys Arg Thr His Thr Gly Glu Lys Lys
50 55 60

Phe Ala Cys Pro Glu Cys Pro Lys Arg Phe Met Gln Ser Cys Asp Leu
65 70 75 80

Thr Arg His Ile Lys Thr His Gln Asn Lys
85 90

<210> 18
<211> 90
<212> PRT
<213> Artificial Sequence

<220>
<223> chemically synthesized zinc finger

<400> 18

Lys Lys Lys Gln His Ile Cys His Ile Gln Gly Cys Gly Lys Val Tyr
1 5 10 15

Gly Asp Arg Ser Asn Leu Thr Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Ser Arg His Lys Arg Thr His Thr Gly Glu Lys Lys
50 55 60

Phe Ala Cys Pro Glu Cys Pro Lys Arg Phe Met Gln Ser Ser Asp Leu
65 70 75 80

Thr Arg His Ile Lys Thr His Gln Asn Lys

85 90

<210> 19
<211> 90
<212> PRT
<213> Artificial Sequence

<220>
<223> chemically synthesized zinc finger

<400> 19

Lys Lys Lys Gln His Ile Cys His Ile Gln Gly Cys Gly Lys Val Tyr
1 5 10 15

Gly Gln Ser Ser Asn Leu Ala Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Asn Leu Ala Arg His Lys Arg Thr His Thr Gly Glu Lys Lys
50 55 60

Phe Ala Cys Pro Glu Cys Pro Lys Arg Phe Met Gln Ser Ser Asp Leu
65 70 75 80

Arg Arg His Ile Lys Thr His Gln Asn Lys
85 90

<210> 20
<211> 90
<212> PRT
<213> Artificial Sequence

<220>
<223> chemically synthesized zinc finger

<400> 20

Lys Lys Lys Gln His Ile Cys His Ile Gln Gly Cys Gly Lys Val Tyr
1 5 10 15

Gly Gln Ser Ser Asn Leu Ala Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Asn Leu Ala Arg His Lys Arg Thr His Thr Gly Glu Lys Lys
50 55 60

Phe Ala Cys Pro Glu Cys Pro Lys Arg Phe Met Gln Ser Ser Asp Leu
65 70 75 80

Thr Arg His Ile Lys Thr His Gln Asn Lys
85 90

<210> 21
<211> 90
<212> PRT
<213> Artificial Sequence

<220>
<223> chemically synthesized zinc finger

<400> 21

Lys Lys Lys Gln His Ile Cys His Ile Gln Gly Cys Gly Lys Val Tyr
1 5 10 15

Gly Asp Arg Ser Asn Leu Thr Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Ser Arg His Lys Arg Thr His Thr Gly Glu Lys Lys
50 55 60

Phe Ala Cys Pro Glu Cys Pro Lys Arg Phe Met Gln Ser Ser Asp Leu
65 70 75 80

Thr Arg His Ile Lys Thr His Gln Asn Lys
85 90

<210> 22

<211> 90
<212> PRT
<213> Artificial Sequence

<220>
<223> chemically synthesized zinc finger

<400> 22

Lys Lys Lys Gln His Ile Cys His Ile Gln Gly Cys Gly Lys Val Tyr
1 5 10 15

Gly Gln Ser Ser Asn Leu Ala Arg His Leu Arg Trp His Thr Gly Glu
20 25 30

Arg Pro Phe Met Cys Thr Trp Ser Tyr Cys Gly Lys Arg Phe Thr Arg
35 40 45

Ser Asp Ala Leu Thr Arg His Lys Arg Thr His Thr Gly Glu Lys Lys
50 55 60

Phe Ala Cys Pro Glu Cys Pro Lys Arg Phe Met Gln Ser Gly Asp Leu
65 70 75 80

Thr Arg His Ile Lys Thr His Gln Asn Lys
85 90

<210> 23
<211> 118
<212> DNA
<213> Artificial Sequence

<220>
<223> chemically synthesized ZFP target sites

<400> 23
ggatccgagc tgtggaatga gagctgtgga atgagagctg tggaatgaga gctgtggaat
60

gagagctgtg gaatgagagc tgtggaatga catatggctg tggaatgtgt gtcagtta
118

<210> 24
<211> 151
<212> DNA
<213> Artificial Sequence

<220>
<223> chemically synthesized ZFP target sites

<400> 24
acgcgttcat tccacagctc tcattccaca gctctcattc cacagctctc attccacagc 60

tctcattcca cagctctcat tccacagctc tcattccaca gctctcattc cacagctctc 120

attccacagc tctcattcca cagtcacgcg t 151

<210> 25
<211> 89
<212> PRT
<213> Artificial Sequence

<220>
<223> chemically synthesized zinc finger motif

<220>
<221> MISC_FEATURE
<222> (2)..(5)
<223> Xaa = any amino acid

<220>
<221> MISC_FEATURE
<222> (4)..(5)
<223> Xaa may be present or absent

<220>
<221> MISC_FEATURE
<222> (7)..(11)
<223> Xaa = any amino acid

<220>
<221> MISC_FEATURE
<222> (13)..(14)
<223> Xaa = any amino acid

<220>
<221> MISC_FEATURE
<222> (16)..(17)
<223> Xaa = any amino acid

<220>
<221> MISC_FEATURE
<222> (20)..(24)
<223> Xaa = any amino acid

<220>
<221> MISC_FEATURE
<222> (23)..(24)
<223> Xaa may be present or absent

<220>
<221> MISC_FEATURE
<222> (26)..(32)
<223> Xaa = any amino acid

<220>
<221> MISC_FEATURE
<222> (34)..(37)
<223> Xaa = any amino acid

<220>
<221> MISC_FEATURE
<222> (36)..(37)
<223> Xaa may be present or absent

<220>
<221> MISC_FEATURE
<222> (39)..(43)
<223> Xaa = any amino acid

<220>
<221> MISC_FEATURE
<222> (45)..(46)
<223> Xaa = any amino acid

<220>
<221> MISC_FEATURE
<222> (48)..(49)
<223> Xaa = any amino acid

<220>
<221> MISC_FEATURE
<222> (52)..(56)
<223> Xaa = any amino acid

<220>
<221> MISC_FEATURE
<222> (55)..(56)
<223> Xaa may be present or absent

<220>
<221> MISC_FEATURE
<222> (58)..(64)
<223> Xaa = any amino acid

<220>
<221> MISC_FEATURE

```
<222>  (66)..(69)
<223>  Xaa = any amino acid

<220>
<221>  MISC_FEATURE
<222>  (68)..(69)
<223>  Xaa may be present or absent

<220>
<221>  MISC_FEATURE
<222>  (71)..(75)
<223>  Xaa = any amino acid

<220>
<221>  MISC_FEATURE
<222>  (77)..(78)
<223>  Xaa = any amino acid

<220>
<221>  MISC_FEATURE
<222>  (80)..(81)
<223>  Xaa = any amino acid

<220>
<221>  MISC_FEATURE
<222>  (84)..(88)
<223>  Xaa = any amino acid

<220>
<221>  MISC_FEATURE
<222>  (87)..(88)
<223>  Xaa may be present or absent

<400>  25

Cys Xaa Xaa Xaa Xaa Cys Xaa Xaa Xaa Xaa Xaa Gln Xaa Xaa Asn Xaa
1               5                   10                  15

Xaa Arg His Xaa Xaa Xaa Xaa Xaa His Xaa Xaa Xaa Xaa Xaa Xaa Xaa
            20                  25                  30

Cys Xaa Xaa Xaa Xaa Cys Xaa Xaa Xaa Xaa Xaa Arg Xaa Xaa Ala Xaa
        35                  40                  45

Xaa Arg His Xaa Xaa Xaa Xaa Xaa His Xaa Xaa Xaa Xaa Xaa Xaa Xaa
    50                  55                  60

Cys Xaa Xaa Xaa Xaa Cys Xaa Xaa Xaa Xaa Xaa Gln Xaa Xaa Asp Xaa
65                  70                  75                  80
```

```
Xaa Arg His Xaa Xaa Xaa Xaa Xaa His
                85
```